# EUROPEAN PATENT APPLICATION

(11) **EP 4 656 723 A1**
(43) Date of publication of application: **03.12.2025**
(21) Application number: 24747374.7
(22) Date of filing: 26.01.2024
(51) Int. Cl.: C12N 15/53, C12N 1/15, C12N 1/19, C12N 1/21, C12N 5/10, C12N 9/02, C12N 15/63, C12Q 1/26

(54) **LUCIFERASE VARIANT**

(30) Priority: 27.01.2023 JP 2023010710
(71) Applicant: Kikkoman Corporation, Noda-shi, Chiba 278-8601 (JP)
(72) Inventor: INAMOTO, Chiaki, Noda-shi, Chiba 278-8601 (JP); ICHIYANAGI, Atsushi, Noda-shi, Chiba 278-8601 (JP)
(74) Representative: Mewburn Ellis LLP
(86) International application number: PCT/JP2024/002442
(87) International publication number: WO 2024/158050

(57) **Abstract**

In an embodiment, the present disclosure provides a firefly luciferase having improved thermostability. In an embodiment, the present disclosure relates to a firefly luciferase mutant comprising an amino acid sequence in which amino acid residues at positions corresponding to positions 252 and the like of SEQ ID NO: 1 are substituted and having improved thermostability, a polynucleotide encoding such luciferase mutant, and a method for producing such luciferase mutant.

## Description

### Technical Field

The present disclosure relates to luciferase mutants having improved thermostability, polynucleotides encoding the luciferase mutants, production methods of the luciferase mutants, kits for detecting at least one of ATP, ADP, and AMP comprising the luciferase mutants, methods for detecting at least one of ATP, ADP, and AMP comprising using the luciferase mutant, and the like.

### Background Art

Firefly luciferase is an enzyme that converts adenosine triphosphate (ATP), D-luciferin, and oxygen into adenosine monophosphate (AMP), oxyluciferin, and carbon dioxide, in the presence of magnesium ions and oxygen, thus generating light. Applying the luminous principle of firefly luciferase allows measuring trace amounts of an enzyme reaction substrate with significantly high sensitivity. Therefore, firefly luciferase has been widely used, for example, for detection of microorganisms in food and beverage materials, assessment of food residue and contamination adhering to fingers and implements, or high-sensitivity measurement methods using various kinds of antibody techniques and gene amplification techniques, or the like, using ATP as the indicator.

However, since coleoptera luciferases, such as firefly luciferases, are generally unstable to heat, there is a drawback that Coleoptera luciferases are susceptible to inactivation when being stored as reagents. Thus, attempts have been made to obtain a luciferase that overcomes this drawback and has satisfactory thermostability.

One attempt is to devise the formulation by adding a salt or the like to the measuring reagent to ensure stability to some extent. However, this method cannot be widely applied due to, for example, constraints of the reagent composition, and has a drawback that the addition of salts is likely to cause some type of reaction impediment in the luciferase reaction.

One of the different attempted approaches besides the devising of the reagent composition is to search for mutant luciferases having preferred properties. For example, Non Patent Literature 1 reports that a North American firefly (Photinus pyralis) luciferase in which the amino acid at position 342 is mutated to alanine is obtained and the luminescence persistency of this firefly luciferase is improved. Additionally, Patent Literature 1 discloses that luciferase of Genji firefly (Luciola cruciata) or Heike firefly (Luciola lateralis) in which the amino acid at position 217 is substituted with a hydrophobic amino acid has heat resistance. Patent Literature 2 discloses that a firefly luciferase having an amino acid sequence in which the amino acid corresponding to position 287 of Heike firefly luciferase is mutated to alanine or the amino acid corresponding to position 392 of Heike firefly luciferase is mutated to isoleucine has improved thermostability.

Patent Literature 3 reports a luciferase mutant or the like in which an amino acid at position 393 of luciferase of Luciola lateralis (SEQ ID NO: 1) is substituted.

Luciferase having high thermostability is needed.

### Citation List

### Patent Literature

Patent Literature 1: JP H05-244942 A (1993)
Patent Literature 2: JP 2011-120559 A
Patent Literature 3: WO 2020/009215
Patent Literature 4: JP 2018-153133 A
Patent Literature 5: WO 1999/014336
Patent Literature 6: WO 2001/020002
Patent Literature 7: WO 2016/070788
Patent Literature 8: WO 2001/31028
Patent Literature 9: WO 2018/071807
Patent Literature 10: WO 2019/236731
Patent Literature 11: JP 2011-188787 A
Patent Literature 12: WO 2019/193391
Patent Literature 13: WO 2007/017684

### Non Patent Literature

Non Patent Literature 1: Biochemistry 2003, No. 42, pp. 10429-10436

### Summary of Invention

### Technical Problem

In a particular embodiment, the technical problem of the present disclosure is to provide a firefly luciferase having improved thermostability. In a particular embodiment, the technical problem of the present disclosure is to provide a firefly luciferase having significantly improved thermostability.

### Solution to the Problem

The present inventors discovered that a firefly luciferase mutant having particular amino acid substitution exhibits improved thermostability, thereby completing the invention of the present application including such discovery as an embodiment. In addition, the present inventors discovered that a firefly luciferase mutant having a plurality of particular amino acid substitutions exhibits significantly improved thermostability, thereby completing the invention of the present application including such discovery as an embodiment.

Accordingly, the present disclosure encompasses the following embodiments.
[1] A luciferase mutant, wherein the luciferase before amino acid substitution has at least 70%, 80%, or 90% amino acid sequence identity to SEQ ID NO: 10, and the luciferase after amino acid substitution has a substitution at the position corresponding to position 252 of SEQ ID NO: 1 with leucine and has improved thermostability, compared to the luciferase before amino acid substitution.
[2] The luciferase mutant of Embodiment 1, wherein the position corresponding to position 262 of SEQ ID NO: 1 is substituted with tyrosine and/or the position corresponding to position 294 of SEQ ID NO: 1 is substituted with leucine.
[3] The luciferase mutant of Embodiment 1 or 2, wherein the amino acid residue at the position corresponding to position 90, 371, 41, 427, 53, 306, or 312 of SEQ ID NO: 1 is substituted.
[4] The luciferase mutant of Embodiment 3, wherein
   (a) the amino acid residue at the position corresponding to position 90 of SEQ ID NO: 1 is substituted and the amino acid residue at the position corresponding to position 90 of SEQ ID NO: 1 is an amino acid residue selected from the group consisting of tyrosine, leucine, isoleucine, cysteine, serine, threonine, aspartic acid, glutamic acid, proline, valine, tryptophan, methionine, alanine, glycine, glutamine, asparagine, lysine, histidine, and arginine;
   (b) the amino acid residue at the position corresponding to position 371 of SEQ ID NO: 1 is substituted and the amino acid residue at the position corresponding to position 371 of SEQ ID NO: 1 is an amino acid residue selected from the group consisting of leucine, tyrosine, cysteine, threonine, aspartic acid, glutamic acid, isoleucine, proline, valine, tryptophan, methionine, alanine, glycine, glutamine, asparagine, lysine, histidine, and arginine;
   (c) the amino acid residue at the position corresponding to position 41 of SEQ ID NO: 1 is substituted and the amino acid residue at the position corresponding to position 41 of SEQ ID NO: 1 is an amino acid residue selected from the group consisting of valine, leucine, isoleucine, methionine, tyrosine, cysteine, serine, threonine, aspartic acid, glutamic acid, tryptophan, phenylalanine, proline, glycine, glutamine, asparagine, lysine, histidine, and arginine;
   (d) the amino acid residue at the position corresponding to position 427 of SEQ ID NO: 1 is substituted and the amino acid residue at the position corresponding to position 427 of SEQ ID NO: 1 is an amino acid residue selected from the group consisting of phenylalanine, leucine, isoleucine, cysteine, serine, threonine, aspartic acid, glutamic acid, tryptophan, methionine, valine, alanine, proline, glycine, glutamine, asparagine, lysine, histidine, and arginine;
   (e) the amino acid residue at the position corresponding to position 53 of SEQ ID NO: 1 is substituted and the amino acid residue at the position corresponding to position 53 of SEQ ID NO: 1 is an amino acid residue selected from the group consisting of leucine, isoleucine, cysteine, serine, threonine, aspartic acid, glutamic acid, tryptophan, methionine, valine, phenylalanine, alanine, proline, glycine, glutamine, asparagine, lysine, histidine, and arginine;
   (f) the amino acid residue at the position corresponding to position 306 of SEQ ID NO: 1 is substituted and the amino acid residue at the position corresponding to position 306 of SEQ ID NO: 1 is an amino acid residue selected from the group consisting of phenylalanine, leucine, isoleucine, cysteine, serine, threonine, aspartic acid, glutamic acid, tryptophan, methionine, valine, alanine, proline, glycine, glutamine, asparagine, lysine, histidine, and arginine; or
   (g) the amino acid residue at the position corresponding to position 312 of SEQ ID NO: 1 is substituted and the amino acid residue at the position corresponding to position 312 of SEQ ID NO: 1 is an amino acid residue selected from the group consisting of tyrosine, phenylalanine, leucine, isoleucine, cysteine, serine, threonine, aspartic acid, glutamic acid, tryptophan, methionine, alanine, proline, glycine, glutamine, asparagine, lysine, histidine, and arginine.
[5] The luciferase mutant of Embodiment 4,
   wherein
   (a) the amino acid residue at the position corresponding to position 90 of SEQ ID NO: 1 is substituted and the amino acid residue at the position corresponding to position 90 of SEQ ID NO: 1 is tyrosine;
   (b) the amino acid residue at the position corresponding to position 371 of SEQ ID NO: 1 is substituted and the amino acid residue at the position corresponding to position 371 of SEQ ID NO: 1 is leucine;
   (c) the amino acid residue at the position corresponding to position 41 of SEQ ID NO: 1 is substituted and the amino acid residue at the position corresponding to position 41 of SEQ ID NO: 1 is valine;
   (d) the amino acid residue at the position corresponding to position 427 of SEQ ID NO: 1 is substituted and the amino acid residue at the position corresponding to position 427 of SEQ ID NO: 1 is phenylalanine;
   (e) the amino acid residue at the position corresponding to position 53 of SEQ ID NO: 1 is substituted and the amino acid residue at the position corresponding to position 53 of SEQ ID NO: 1 is leucine;
   (f) the amino acid residue at the position corresponding to position 306 of SEQ ID NO: 1 is substituted and the amino acid residue at the position corresponding to position 306 of SEQ ID NO: 1 is phenylalanine; or
   (g) the amino acid residue at the position corresponding to position 312 of SEQ ID NO: 1 is substituted and the amino acid residue at the position corresponding to position 312 of SEQ ID NO: 1 is glutamic acid, lysine, or arginine.
[6] The luciferase mutant of any of Embodiments 1 to 5, which further comprises an amino acid sequence in which the amino acid residue at the position corresponding to position 221, 17, 18, 75, 110, 223, 224, 257, 229, or 158 of SEQ ID NO: 1 is substituted.
[7] The luciferase mutant of Embodiment 6,
   wherein
   (i) the amino acid residue at the position corresponding to position 221 of SEQ ID NO: 1 is substituted and the amino acid residue at the position corresponding to position 221 of SEQ ID NO: 1 is an amino acid residue selected from the group consisting of leucine, tyrosine, cysteine, serine, threonine, aspartic acid, glutamic acid, isoleucine, proline, valine, tryptophan, methionine, alanine, glycine, glutamine, asparagine, lysine, histidine, and arginine;
   (ii) the amino acid residue at the position corresponding to position 17 of SEQ ID NO: 1 is substituted and the amino acid residue at the position corresponding to position 17 of SEQ ID NO: 1 is an amino acid residue selected from the group consisting of aspartic acid, glutamic acid, lysine, histidine, arginine, tyrosine, cysteine, serine, threonine, tryptophan, methionine, leucine, isoleucine, valine, alanine, proline, glycine, glutamine, and asparagine;
   (iii) the amino acid residue at the position corresponding to position 18 of SEQ ID NO: 1 is substituted and the amino acid residue at the position corresponding to position 18 of SEQ ID NO: 1 is an amino acid residue selected from the group consisting of leucine, isoleucine, cysteine, serine, threonine, aspartic acid, glutamic acid, tryptophan, methionine, valine, phenylalanine, alanine, proline, glycine, glutamine, asparagine, lysine, histidine, and arginine;
   (iv) the amino acid residue at the position corresponding to position 75 of SEQ ID NO: 1 is substituted and the amino acid residue at the position corresponding to position 75 of SEQ ID NO: 1 is an amino acid residue selected from the group consisting of lysine, histidine, arginine, aspartic acid, glutamic acid, tyrosine, cysteine, serine, threonine, tryptophan, methionine, leucine, isoleucine, phenylalanine, alanine, proline, glycine, glutamine, and asparagine;
   (v) the amino acid residue at the position corresponding to position 110 of SEQ ID NO: 1 is substituted and the amino acid residue at the position corresponding to position 110 of SEQ ID NO: 1 is an amino acid residue selected from the group consisting of aspartic acid, glutamic acid, lysine, arginine, histidine, tyrosine, cysteine, serine, threonine, tryptophan, methionine, leucine, valine, phenylalanine, alanine, proline, glycine, glutamine, and asparagine;
   (vi) the amino acid residue at the position corresponding to position 223 of SEQ ID NO: 1 is substituted and the amino acid residue at the position corresponding to position 223 of SEQ ID NO: 1 is an amino acid residue selected from the group consisting of valine, isoleucine, leucine, tyrosine, cysteine, serine, threonine, aspartic acid, glutamic acid, tryptophan, methionine, phenylalanine, alanine, proline, glycine, glutamine, asparagine, lysine, and arginine;
   (vii) the amino acid residue at the position corresponding to position 224 of SEQ ID NO: 1 is substituted and the amino acid residue at the position corresponding to position 224 of SEQ ID NO: 1 is an amino acid residue selected from the group consisting of isoleucine, leucine, valine, methionine, cysteine, tyrosine, serine, threonine, aspartic acid, glutamic acid, tryptophan, phenylalanine, proline, glycine, glutamine, asparagine, lysine, histidine, and arginine;
   (viii) the amino acid residue at the position corresponding to position 257 of SEQ ID NO: 1 is substituted and the amino acid residue at the position corresponding to position 257 of SEQ ID NO: 1 is an amino acid residue selected from the group consisting of phenylalanine, leucine, isoleucine, cysteine, serine, threonine, aspartic acid, glutamic acid, tryptophan, methionine, valine, alanine, proline, glycine, glutamine, asparagine, lysine, histidine, and arginine;
   (ix) the amino acid residue at the position corresponding to position 229 of SEQ ID NO: 1 is substituted and the amino acid residue at the position corresponding to position 229 of SEQ ID NO: 1 is an amino acid residue selected from the group consisting of phenylalanine, leucine, isoleucine, cysteine, serine, threonine, aspartic acid, glutamic acid, tryptophan, methionine, valine, alanine, proline, glycine, glutamine, asparagine, lysine, histidine, and arginine; or
   (x) the amino acid residue at the position corresponding to position 158 of SEQ ID NO: 1 is substituted and the amino acid residue at the position corresponding to position 158 of SEQ ID NO: 1 is an amino acid residue selected from the group consisting of aspartic acid, glutamic acid, lysine, arginine, histidine, tyrosine, cysteine, serine, threonine, tryptophan, methionine, leucine, isoleucine, phenylalanine, alanine, proline, glycine, glutamine, and asparagine.
[8] The luciferase mutant of Embodiment 7,
   wherein
   (i) the amino acid residue at the position corresponding to position 221 of SEQ ID NO: 1 is substituted and the amino acid residue at the position corresponding to position 221 of SEQ ID NO: 1 is leucine;
   (ii) the amino acid residue at the position corresponding to position 17 of SEQ ID NO: 1 is substituted and the amino acid residue at the position corresponding to position 17 of SEQ ID NO: 1 is aspartic acid, glutamic acid, or lysine;
   (iii) the amino acid residue at the position corresponding to position 18 of SEQ ID NO: 1 is substituted and the amino acid residue at the position corresponding to position 18 of SEQ ID NO: 1 is leucine;
   (iv) the amino acid residue at the position corresponding to position 75 of SEQ ID NO: 1 is substituted and the amino acid residue at the position corresponding to position 75 of SEQ ID NO: 1 is lysine;
   (v) the amino acid residue at the position corresponding to position 110 of SEQ ID NO: 1 is substituted and the amino acid residue at the position corresponding to position 110 of SEQ ID NO: 1 is aspartic acid, glutamic acid, lysine, or arginine;
   (vi) the amino acid residue at the position corresponding to position 223 of SEQ ID NO: 1 is substituted and the amino acid residue at the position corresponding to position 223 of SEQ ID NO: 1 is valine, isoleucine, or leucine;
   (vii) the amino acid residue at the position corresponding to position 224 of SEQ ID NO: 1 is substituted and the amino acid residue at the position corresponding to position 224 of SEQ ID NO: 1 is isoleucine;
   (viii) the amino acid residue at the position corresponding to position 257 of SEQ ID NO: 1 is substituted and the amino acid residue at the position corresponding to position 257 of SEQ ID NO: 1 is phenylalanine;
   (ix) the amino acid residue at the position corresponding to position 229 of SEQ ID NO: 1 is substituted and the amino acid residue at the position corresponding to position 229 of SEQ ID NO: 1 is phenylalanine or leucine; or
   (x) the amino acid residue at the position corresponding to position 158 of SEQ ID NO: 1 is substituted and the amino acid residue at the position corresponding to position 158 of SEQ ID NO: 1 is aspartic acid or glutamic acid.
[9] The luciferase mutant of any of Embodiments 1 to 8, wherein the luciferase before amino acid substitution comprises an amino acid sequence selected from the group consisting of (A) to (F):
   (A) an amino acid sequence having at least 70%, 80%, or 90% amino acid sequence identity to SEQ ID NO: 10;
   (B) with regard to the amino acid sequence of (A), additionally, sequence identity between the homologous region of SEQ ID NO: 1 and the homologous region of the luciferase before amino acid substitution is 90% or higher in the amino acid sequence;
   (C) an amino acid sequence derived from the amino acid sequence of SEQ ID NO: 10 by substitution, deletion, or addition of one or several amino acids at a position other than the position corresponding to position 252 of SEQ ID NO: 1;
   (D) with regard to the amino acid sequence of (C), additionally, an amino acid sequence derived from the amino acid sequence of SEQ ID NO: 1, 10, 69, 71, or 73 by substitution, deletion, or addition of one or several amino acids at positions other than positions corresponding to position 90, 262, 371, 41, 427, 53, 306, 312, 294, 221, 17, 18, 75, 110, 223, 224, 257, 229, or 158 of SEQ ID NO: 1;
   (E) the amino acid sequence of SEQ ID NO: 10; and
   (F) an amino acid sequence of luciferase of Luciola lateralis.
[10] The luciferase mutant of Embodiment 9, wherein the luciferase before amino acid substitution comprises an amino acid sequence selected from the group consisting of (a) to (d):
   (a) an amino acid sequence having 90% or higher amino acid sequence identity to SEQ ID NO: 10;
   (b) an amino acid sequence derived from the amino acid sequence of SEQ ID NO: 1 by substitution, deletion, or addition of one or several amino acids at a position other than position corresponding to position 252 of SEQ ID NO: 1;
   (c) with regard to the amino acid sequence of (b), additionally, an amino acid sequence derived from the amino acid sequence of SEQ ID NO: 1 by substitution, deletion, or addition of one or several amino acids at positions other than positions corresponding to position 262, 371, 41, 427, 53, 306, 312, 252, 294, 221, 17, 18, 75, 110, 223, 224, 257, 229, or 158 of SEQ ID NO: 1; and
   (d) the amino acid sequence of SEQ ID NO: 10 .
[11] A polynucleotide encoding the luciferase mutant of any one of Embodiments 1 to 10.
[12] A vector comprising the polynucleotide of Embodiment 11.
[13] A host cell comprising the polynucleotide of Embodiment 11 or the vector of Embodiment 12.
[14] A production method of a luciferase mutant having improved thermostability comprising a step of culturing the host cell of Embodiment 13.
[15] A kit for detecting at least one of ATP, ADP, and AMP comprising the luciferase mutant of any of Embodiments 1 to 10.
[16] A method for detecting at least one of ATP, ADP, and AMP comprising using the luciferase mutant of any of Embodiments 1 to 10.
[17] A luciferase mutant comprising an amino acid sequence in which at least one amino acid residue at the position corresponding to position 252, 262, 294, 90, 371, 41, 427, 53, 306, 312, 221, 17, 18, 75, 110, 223, 224, 257, 229, or 158 of SEQ ID NO: 1 is substituted, wherein the luciferase after amino acid substitution has improved thermostability compared with that of the luciferase before amino acid substitution.
[18] The luciferase mutant of Embodiment 17,
   wherein
   the position corresponding to position 252 of SEQ ID NO: 1 is substituted with leucine;
   the position corresponding to position 262 of SEQ ID NO: 1 is substituted with tyrosine;
   the position corresponding to position 294 of SEQ ID NO: 1 is substituted with leucine;
   the position corresponding to position 90 of SEQ ID NO: 1 is substituted with tyrosine;
   the position corresponding to position 371 of SEQ ID NO: 1 is substituted with leucine;
   the position corresponding to position 41 of SEQ ID NO: 1 is substituted with valine;
   the position corresponding to position 427 of SEQ ID NO: 1 is substituted with phenylalanine;
   the position corresponding to position 53 of SEQ ID NO: 1 is substituted with leucine;
   the position corresponding to position 306 of SEQ ID NO: 1 is substituted with phenylalanine;
   the position corresponding to position 312 of SEQ ID NO: 1 is substituted with glutamic acid, lysine, or arginine;
   the position corresponding to position 221 of SEQ ID NO: 1 is substituted with leucine;
   the position corresponding to position 17 of SEQ ID NO: 1 is substituted with aspartic acid, glutamic acid, or lysine;
   the position corresponding to position 18 of SEQ ID NO: 1 is substituted with leucine;
   the position corresponding to position 75 of SEQ ID NO: 1 is substituted with lysine;
   the position corresponding to position 110 of SEQ ID NO: 1 is substituted with aspartic acid, glutamic acid, lysine, or arginine;
   the position corresponding to position 223 of SEQ ID NO: 1 is substituted with valine, isoleucine, or leucine;
   the position corresponding to position 224 of SEQ ID NO: 1 is substituted with isoleucine;
   the position corresponding to position 257 of SEQ ID NO: 1 is substituted with phenylalanine;
   the position corresponding to position 229 of SEQ ID NO: 1 is substituted with phenylalanine or leucine; or
   the position corresponding to position 158 of SEQ ID NO: 1 is substituted with aspartic acid or glutamic acid.

The present description encompasses the disclosed contents of Japanese Patent Application No. 2023-010710, which is the basis of the priority of the present application. Advantageous Effects of Invention

The present disclosure provides a firefly luciferase having improved thermostability.

### Brief Description of Drawings

Figure 1 illustrates alignment results of wild-type luciferases of Luciola lateralis, Luciola cruciata, Photinus pyralis, and Photuris pennsylvanica. In the drawing, the same amino acid residues in four amino acid sequences are enclosed by frames.
Figure 2 continues from Figure 1.

### Description of Embodiments

### (Reference sequence)

In the present description, positions in a sequence are defined by designating SEQ ID NO: 1 as the reference sequence, unless otherwise specified. SEQ ID NO: 1 is an amino acid sequence of luciferase derived from Luciola lateralis. SEQ ID NO: 10 is a mutant prepared based on SEQ ID NO: 1. SEQ ID NO: 10 is derived from SEQ ID NO: 1 by amino acid substitutions A217L, E490K, C393L, G326S, V392I, and F467I.

### (Luciferase mutant)

In an embodiment, the present disclosure relates to a firefly luciferase mutant comprising an amino acid sequence in which the amino acid residue at the position corresponding to positions 252, 262, 294, 90, 371, 41, 427, 53, 306, and/or 312 of SEQ ID NO: 1 is substituted (such positions may be referred to as "position 252 and the like" herein). In an embodiment, the present disclosure further relates to a firefly luciferase mutant comprising an amino acid sequence in which the amino acid residue at the position corresponding to positions 221, 17, 18, 75, 110, 223, 224, 257, 229, and/or 158 of SEQ ID NO: 1 is substituted. In an embodiment, such position may be substituted with any of 19 types of amino acids other than the amino acid residue in a wild-type sequence. In an embodiment, the present disclosure relates to a firefly luciferase mutant having at least one amino acid substitution corresponding to amino acid substitutions F252L, F262Y, F294L, F90Y, F371L, A41V, Y427F, Y53L, Y306F, F221L, F17(D, E, K), Y18L, V75K, I110(D, E, K, R), H223(V, I, L), A224I, Y257F, Y229(F, L), and V158(D, E) in SEQ ID NO: 1. In an embodiment, a luciferase mutant can have improved thermostability, compared with that of the luciferase before amino acid substitution.

In the present description, "wild-type" refers to a trait present the most in nature in a conspecific group.

Firefly luciferase derived from any firefly can be used as the firefly luciferase. For example, Luciola lateralis, Luciola cruciata, Photinus pyralis, Photuris pennsylvanica, Lampyris noctiluca, Pyrocoelia miyako, Pyrophorus plagiophthalamus, or Luciola mingrelica, preferably the firefly luciferase from Luciola lateralis, Luciola cruciata, Photinus pyralis, or Photuris pennsylvanica can be used. Alternatively, chimeric proteins produced based on luciferase genes derived from various kinds of fireflies may be used.

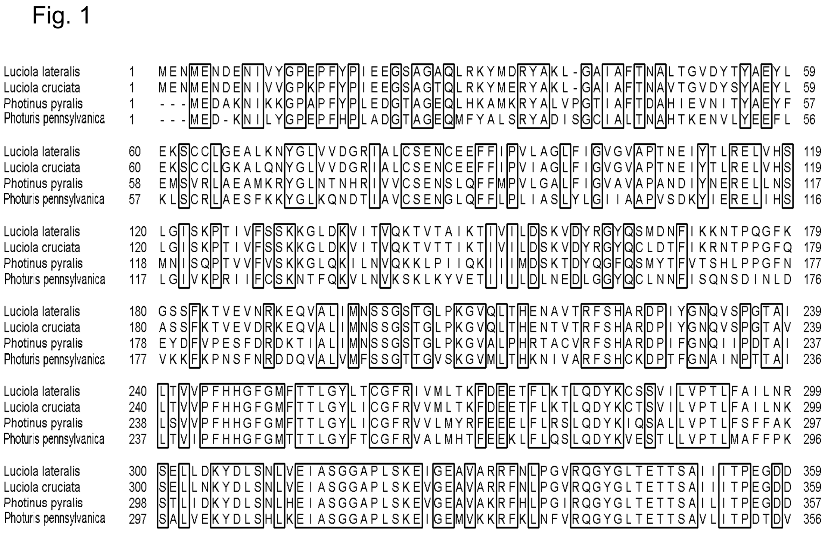

In the present description, a correspondence relationship of amino acid positions can readily be identified through comparison of amino acid sequences of various kinds of firefly luciferases using, for example, the existing software for homology analysis of amino acids, for example, GENETYX (manufactured by GENETYX CORPORATION) or the like. For example, the amino acid position of a luciferase corresponding to position X in the amino acid sequence of SEQ ID NO: 1 can be identified by aligning the amino acid sequence of the luciferase with the amino acid sequence of SEQ ID NO: 1. For example, the "position corresponding to position 262 of SEQ ID NO: 1" may be position 262 in the amino acid sequence of SEQ ID NO 69, position 260 of SEQ ID NO 71, or position 259 of SEQ ID NO 73. Figure 1 illustrates alignment results of Luciola lateralis luciferase, Luciola cruciata luciferase, Photinus pyralis luciferase, and Photuris pennsylvanica luciferase. A corresponding position in each amino acid sequence can be determined with reference to such alignment results.

In an embodiment, the amino acid at the position corresponding to position 252 of SEQ ID NO: 1 in the luciferase mutant is a non-acidic amino acid other than phenylalanine (for example, leucine, tyrosine, isoleucine, cysteine, serine, threonine, aspartic acid, glutamic acid, tryptophan, methionine, valine, alanine, proline, glycine, glutamine, asparagine, lysine, histidine, or arginine). Substitution (mutation) of phenylalanine at position 252 of SEQ ID NO: 1 with leucine may be referred to as "F252L" herein. It should be noted that a target of F252L introduction is not limited to SEQ ID NO: 1, and F252L may be introduced into a position corresponding to position 252 of SEQ ID NO: 1 of any luciferase. Depending on the luciferase into which F252L is to be introduced, the position corresponding to position 252 of SEQ ID NO: 1 before substitution need not necessarily be F. In other words, "F252L" is merely a notation for convenience relative to SEQ ID NO: 1. As long as L is introduced into position corresponding to position 252 of SEQ ID NO: 1, such amino acid substitution amounts to (is equivalent to) F252L. However, a sequence of a wild-type luciferase in which the position corresponding to position 252 of SEQ ID NO: 1 is already L (i.e., L252L) is excluded from mutation of the present disclosure. In other words, wild-type sequences are excluded from the present disclosure, and natural sequences are excluded as well. Other amino acid substitution at other position may be denoted in the same manner (amino acid before substitution - position - amino acid after substitution). In an embodiment, a luciferase mutant may comprise an amino acid sequence having high sequence identity, for example, 70% or higher, 75% or higher, 80% or higher, 85% or higher, 90% or higher, 91% or higher, 92% or higher, 93% or higher, 94% or higher, 95% or higher, 96% or higher, 97% or higher, 98% or higher, or 99% or higher sequence identity to the amino acid sequence of SEQ ID NO: 10. In an embodiment, luciferase before amino acid substitution may be luciferase derived from Luciola lateralis or luciferase derived from Luciola cruciata.

In an embodiment, the mutation at the position corresponding to position 252 and the like of SEQ ID NO: 1 is artificially introduced. This can be achieved by artificially introducing the mutation in a sequence of a gene encoding luciferase.

In an embodiment, the firefly luciferase mutant may further comprise a mutation other than the mutation at position 252 and the like of SEQ ID NO: 1. The mutation may be artificially introduced intending some specific effect or may be randomly or non-artificially introduced. Examples of mutations introduced with the purpose (intention) of obtaining a specific effect include addition, deletion, or modification of the sequence to enhance the firefly luciferase gene expression level, addition, deletion, or modification of the sequence to improve purification efficiency of the firefly luciferase, and various kinds of mutations that give a practically preferred property to firefly luciferases. Examples of such publicly known mutations include mutations that enhance luminescence persistency as described in JP 2000-197484 A, mutations that change the emission wavelength as described in JP H03-285683 A (1991) or JP 2003-512071 T, mutations that enhance surfactant resistance as described in JP H11-239493 A (1999), mutations that change substrate affinity as described in WO 99/02697, JP H10-512750 T (1998), or JP 2001-518799 T, mutations that enhance stability as described in JP H05-244942 A (1993), JP 2011-120559 A, JP 2000-197487 A, JP H09-510610 T (1997), or JP 2003-518912 T, and mutations that improve luminescence persistency, stability, and luminescence amount as described in JP 2011-188787 A. For example, JP 2011-120559 A discloses that thermostability is improved in firefly luciferase having an amino acid sequence in which the amino acid corresponding to position 287 of Luciola lateralis luciferase is mutated to alanine or the amino acid corresponding to position 392 thereof is mutated to isoleucine. JP 2011-120559 A describes that combinations of these mutations, a mutation in which the amino acid at position 326 is substituted with serine, and/or a mutation in which the amino acid at position 467 is substituted with isoleucine allow obtaining firefly luciferases with further improved stability. For example, WO 2020/009215 discloses a thermostable luciferase mutant in which position 393 of SEQ ID NO: 1 is substituted.

In one embodiment, with regard to the luciferase mutant of the present disclosure,
(a) the amino acid residue at the position corresponding to position 252 of SEQ ID NO: 1 is an amino acid residue selected from the group consisting of leucine, tyrosine, cysteine, serine, threonine, aspartic acid, glutamic acid, isoleucine, proline, valine, tryptophan, alanine, glycine, glutamine, asparagine, lysine, histidine, arginine, and methionine, for example leucine.
   In one embodiment, with regard to the luciferase mutant of the present disclosure,
(b) the amino acid residue at the position corresponding to position 262 of SEQ ID NO: 1 is substituted and the amino acid residue at the position corresponding to position 262 of SEQ ID NO: 1 is an amino acid residue selected from the group consisting of tyrosine, leucine, isoleucine, cysteine, serine, threonine, aspartic acid, glutamic acid, tryptophan, methionine, valine, alanine, proline, glycine, glutamine, asparagine, lysine, histidine, and arginine, for example tyrosine.
   In one embodiment, with regard to the luciferase mutant of the present disclosure,
(c) the amino acid residue at the position corresponding to position 294 of SEQ ID NO: 1 is an amino acid residue selected from the group consisting of leucine, tyrosine, cysteine, serine, threonine, aspartic acid, glutamic acid, isoleucine, proline, valine, tryptophan, methionine, alanine, glycine, glutamine, asparagine, lysine, histidine, and arginine, for example leucine.

In one embodiment, with regard to the luciferase mutant of the present disclosure, in addition:
(a) the amino acid residue at the position corresponding to position 90 of SEQ ID NO: 1 is substituted and the amino acid residue at the position corresponding to position 90 of SEQ ID NO: 1 is an amino acid residue selected from the group consisting of tyrosine, leucine, isoleucine, cysteine, serine, threonine, aspartic acid, glutamic acid, proline, valine, tryptophan, methionine, alanine, glycine, glutamine, asparagine, lysine, histidine, and arginine, for example tyrosine;
(b) the amino acid residue at the position corresponding to position 371 of SEQ ID NO: 1 is substituted and the amino acid residue at the position corresponding to position 371 of SEQ ID NO: 1 is an amino acid residue selected from the group consisting of leucine, tyrosine, cysteine, threonine, aspartic acid, glutamic acid, isoleucine, proline, valine, tryptophan, methionine, alanine, glycine, glutamine, asparagine, lysine, histidine, and arginine, for example leucine;
(c) the amino acid residue at the position corresponding to position 41 of SEQ ID NO: 1 is substituted and the amino acid residue at the position corresponding to position 41 of SEQ ID NO: 1 is an amino acid residue selected from the group consisting of valine, leucine, isoleucine, methionine, tyrosine, cysteine, serine, threonine, aspartic acid, glutamic acid, tryptophan, phenylalanine, proline, glycine, glutamine, asparagine, lysine, histidine, and arginine, for example valine;
(d) the amino acid residue at the position corresponding to position 427 of SEQ ID NO: 1 is substituted and the amino acid residue at the position corresponding to position 427 of SEQ ID NO: 1 is an amino acid residue selected from the group consisting of phenylalanine, leucine, isoleucine, cysteine, serine, threonine, aspartic acid, glutamic acid, tryptophan, methionine, valine, alanine, proline, glycine, glutamine, asparagine, lysine, histidine, and arginine, for example phenylalanine;
(e) the amino acid residue at the position corresponding to position 53 of SEQ ID NO: 1 is substituted and the amino acid residue at the position corresponding to position 53 of SEQ ID NO: 1 is an amino acid residue selected from the group consisting of leucine, isoleucine, cysteine, serine, threonine, aspartic acid, glutamic acid, tryptophan, methionine, valine, phenylalanine, alanine, proline, glycine, glutamine, asparagine, lysine, histidine, and arginine, for example leucine;
(f) the amino acid residue at the position corresponding to position 306 of SEQ ID NO: 1 is substituted and the amino acid residue at the position corresponding to position 306 of SEQ ID NO: 1 is an amino acid residue selected from the group consisting of phenylalanine, leucine, isoleucine, cysteine, serine, threonine, aspartic acid, glutamic acid, tryptophan, methionine, valine, alanine, proline, glycine, glutamine, asparagine, lysine, histidine, and arginine, for example phenylalanine; or
(g) the amino acid residue at the position corresponding to position 312 of SEQ ID NO: 1 is substituted and the amino acid residue at the position corresponding to position 312 of SEQ ID NO: 1 is an amino acid residue selected from the group consisting of tyrosine, phenylalanine, leucine, isoleucine, cysteine, serine, threonine, aspartic acid, glutamic acid, tryptophan, methionine, alanine, proline, glycine, glutamine, asparagine, lysine, histidine, and arginine.

### (Further amino acid substitution(s))

In one embodiment, with regard to the luciferase mutant of the present disclosure, the amino acid residue at the position corresponding to position(s) 221, 17, 18, 75, 110, 223, 224, 257, 229, and/or 158 of SEQ ID NO: 1 is substituted, and luciferase after amino acid substitution can have improved thermostability, compared with that of the luciferase before amino acid substitution.

In an embodiment, a luciferase mutant having a further amino acid substitution may have at least one of the following amino acid substitutions:
(i) the amino acid residue at the position corresponding to position 221 of SEQ ID NO: 1 is substituted and the amino acid residue at the position corresponding to position 221 of SEQ ID NO: 1 is an amino acid residue selected from the group consisting of leucine, tyrosine, cysteine, serine, threonine, aspartic acid, glutamic acid, isoleucine, proline, valine, tryptophan, methionine, alanine, glycine, glutamine, asparagine, lysine, histidine, and arginine, for example leucine;
(ii) the amino acid residue at the position corresponding to position 17 of SEQ ID NO: 1 is substituted and the amino acid residue at the position corresponding to position 17 of SEQ ID NO: 1 is an amino acid residue selected from the group consisting of aspartic acid, glutamic acid, lysine, histidine, arginine, tyrosine, cysteine, serine, threonine, tryptophan, methionine, leucine, isoleucine, valine, alanine, proline, glycine, glutamine, and asparagine, for example aspartic acid, glutamic acid, or lysine;
(iii) the amino acid residue at the position corresponding to position 18 of SEQ ID NO: 1 is substituted and the amino acid residue at the position corresponding to position 18 of SEQ ID NO: 1 is an amino acid residue selected from the group consisting of leucine, isoleucine, cysteine, serine, threonine, aspartic acid, glutamic acid, tryptophan, methionine, valine, phenylalanine, alanine, proline, glycine, glutamine, asparagine, lysine, histidine, and arginine, for example leucine;
(iv) the amino acid residue at the position corresponding to position 75 of SEQ ID NO: 1 is substituted and the amino acid residue at the position corresponding to position 75 of SEQ ID NO: 1 is an amino acid residue selected from the group consisting of lysine, histidine, arginine, aspartic acid, glutamic acid, tyrosine, cysteine, serine, threonine, tryptophan, methionine, leucine, isoleucine, phenylalanine, alanine, proline, glycine, glutamine, and asparagine, for example lysine;
(v) the amino acid residue at the position corresponding to position 110 of SEQ ID NO: 1 is substituted and the amino acid residue at the position corresponding to position 110 of SEQ ID NO: 1 is an amino acid residue selected from the group consisting of aspartic acid, glutamic acid, lysine, arginine, histidine, tyrosine, cysteine, serine, threonine, tryptophan, methionine, leucine, valine, phenylalanine, alanine, proline, glycine, glutamine, and asparagine, for example aspartic acid, glutamic acid, lysine, or arginine;
(vi) the amino acid residue at the position corresponding to position 223 of SEQ ID NO: 1 is substituted and the amino acid residue at the position corresponding to position 223 of SEQ ID NO: 1 is an amino acid residue selected from the group consisting of valine, isoleucine, leucine, tyrosine, cysteine, serine, threonine, aspartic acid, glutamic acid, tryptophan, methionine, phenylalanine, alanine, proline, glycine, glutamine, asparagine, lysine, and arginine, for example valine, isoleucine, or leucine;
(vii) the amino acid residue at the position corresponding to position 224 of SEQ ID NO: 1 is substituted and the amino acid residue at the position corresponding to position 224 of SEQ ID NO: 1 is an amino acid residue selected from the group consisting of isoleucine, leucine, valine, methionine, cysteine, tyrosine, serine, threonine, aspartic acid, glutamic acid, tryptophan, phenylalanine, proline, glycine, glutamine, asparagine, lysine, histidine, and arginine, for example isoleucine;
(viii) the amino acid residue at the position corresponding to position 257 of SEQ ID NO: 1 is substituted and the amino acid residue at the position corresponding to position 257 of SEQ ID NO: 1 is an amino acid residue selected from the group consisting of phenylalanine, leucine, isoleucine, cysteine, serine, threonine, aspartic acid, glutamic acid, tryptophan, methionine, valine, alanine, proline, glycine, glutamine, asparagine, lysine, histidine, and arginine, for example phenylalanine;
(ix) the amino acid residue at the position corresponding to position 229 of SEQ ID NO: 1 is substituted and the amino acid residue at the position corresponding to position 229 of SEQ ID NO: 1 is an amino acid residue selected from the group consisting of phenylalanine, leucine, isoleucine, cysteine, serine, threonine, aspartic acid, glutamic acid, tryptophan, methionine, valine, alanine, proline, glycine, glutamine, asparagine, lysine, histidine, and arginine, for example phenylalanine or leucine; and/or
(x) the amino acid residue at the position corresponding to position 158 of SEQ ID NO: 1 is substituted and the amino acid residue at the position corresponding to position 158 of SEQ ID NO: 1 is an amino acid residue selected from the group consisting of aspartic acid, glutamic acid, lysine, arginine, histidine, tyrosine, cysteine, serine, threonine, tryptophan, methionine, leucine, isoleucine, phenylalanine, alanine, proline, glycine, glutamine, and asparagine, for example aspartic acid or glutamic acid.

### (Double mutant)

In an embodiment, the luciferase mutant of the present disclosure may comprise a double mutation selected from the group consisting of F252L/F262Y, F262Y/F294L, and F252L/F294L in SEQ ID NO: 1 as the reference sequence.

### (Triple mutant)

In an embodiment, the luciferase mutant of the present disclosure may comprise a triple mutation F252L/F262Y/F294L in SEQ ID NO: 1 as the reference sequence. Into such triple mutant, at least one of the amino acid substitutions of the present disclosure can further be introduced. A triple mutant may comprise 90% or higher amino acid sequence identity to SEQ ID NO: 1, 2, or 10. In one embodiment, when a triple mutant is heat-treated at 60°C for 5 minutes, residual activity after heat treatment is improved by at least 2 times, 3 times, 4 times, or 5 times, compared with that of the luciferase before introduction of 3 amino acid substitutions. In one embodiment, when a triple mutant is heat-treated at 60°C for 5 minutes, residual activity after heat treatment is improved by at least 100%, 200%, 300%, or 400%, compared with that of the luciferase before introduction of 3 amino acid substitutions. The phrase "firefly luciferase having significantly improved thermostability" used herein refers to a firefly luciferase having residual activity after heat treatment that is improved by at least 100%, 200%, 300%, or 400%, compared with that of the reference firefly luciferase before mutation, when it is heat-treated at 60°C for 5 minutes.

In an embodiment, luciferase before amino acid substitution may comprise an amino acid sequence selected from the group consisting of (A) to (F) below:
(A) an amino acid sequence having at least 70%, 75%, 80%, 85%, 90%, 91%, 92%, 93%, 94%, 95%, 96%, 97%, 98%, or 99% amino acid sequence identity to SEQ ID NO: 1, 2, or 10;
(B) with regard to the amino acid sequence of (A), additionally, an amino acid sequence in which sequence identity between the homologous region of SEQ ID NO: 1 and the homologous region of the luciferase before amino acid substitution is at least 70%, 75%, 80%, 85%, 90%, 91%, 92%, 93%, 94%, 95%, 96%, 97%, 98%, or 99% or higher;
(C) an amino acid sequence derived from the amino acid sequence of SEQ ID NO: 1 or 10 by substitution, deletion, or addition of one or several amino acids at a position other than position corresponding to position 252 of SEQ ID NO: 1;
(D) with regard to the amino acid sequence of (C), additionally, an amino acid sequence derived from the amino acid sequence of SEQ ID NO: 1 or 10 by substitution, deletion, or addition of one or several amino acids at positions other than positions corresponding to position 90, 262, 371, 41, 427, 53, 306, 312, 294, 221, 17, 18, 75, 110, 223, 224, 257, 229, or 158 of SEQ ID NO: 1;
(E) the amino acid sequence of SEQ ID NO: 1, 2, or 10; and
(F) an amino acid sequence of luciferase of Luciola lateralis or an amino acid sequence of luciferase of Luciola cruciata.

Homologous regions can be identified as regions in which identical amino acid residues are conserved in the 4 types of firefly luciferases shown in Figure 1 (i.e., Luciola lateralis, Luciola cruciata, Photinus pyralis, and Photuris pennsylvanica). Unless otherwise specified, in the present description, a homologous region of SEQ ID NO: 1 is a region of the amino acid sequence consisting of all of the amino acids at positions indicated below in SEQ ID NO: 1: positions 4 to 5, positions 9 to 10, positions 13 to 14, positions 16 to 17, position 19, position 23, positions 25 to 26, position 28, positions 35 to 37, position 40, positions 42 to 43, position 45, position 47, position 55, position 57, position 62, position 65, positions 72 to 74, position 80, positions 83 to 86, positions 90 to 91, position 93, position 98, position 101, positions 105 to 106, position 111, positions 114 to 116, position 119, position 122, position 125, position 129, positions 131 to 132, position 137, position 141, position 151, position 153, position 155, position 159, position 162, position 164, position 169, position 183, position 190, positions 195 to 196, position 198, positions 200 to 202, positions 204 to 205, positions 208 to 210, position 212, position 214, positions 220 to 223, positions 226 to 227, positions 230 to 231, position 235, positions 237 to 238, position 240, position 242, positions 244 to 251, positions 253 to 257, positions 260 to 263, position 270, position 272, positions 275 to 276, positions 279 to 283, position 286, positions 289 to 293, position 300, position 302, positions 305 to 309, position 311, positions 313 to 324, positions 326 to 327, position 329, positions 332 to 333, position 335, positions 339 to 350, positions 353 to 355, position 358, positions 361 to 362, positions 365 to 366, positions 368 to 369, positions 374 to 375, position 377, position 380, position 382, positions 384 to 385, position 387, positions 390 to 391, position 396, position 398, position 400, position 403, position 406, positions 408 to 410, position 414, positions 418 to 420, positions 423 to 425, position 427, position 429, positions 433 to 434, positions 436 to 451, positions 453 to 455, position 457, positions 460 to 464, position 466, positions 468 to 471, position 473, positions 475 to 476, positions 479 to 483, position 485, positions 487 to 488, positions 492 to 493, position 497, position 504, positions 506 to 508, positions 511 to 512, positions 514 to 518, position 520, positions 522 to 523, positions 525 to 527, positions 529 to 531, position 533, position 538, position 543, and position 547. Unless otherwise specified, in the present description, a homologous region of a target firefly luciferase is a region corresponding to the homologous region of SEQ ID NO: 1. When comparing homologous regions with each other, a position in one homologous region corresponds to the same position in the other homologous region. For example, when comparing a homologous region of Sequence A with a homologous region of Sequence B, the position corresponding to position 4 of SEQ ID NO: 1 in Sequence A is compared with the position corresponding to position 4 of SEQ ID NO: 1 in Sequence B. A homologous region is defined on the basis of the sequence before amino acid substitution as the reference sequence and does not prevent introduction of a mutation of a luciferase into a homologous region. For example, position 262 of SEQ ID NO: 1 is included in the homologous region of SEQ ID NO: 1, and mutation may be introduced into this position. The same applies to other sites of mutation described herein.

Amino acid sequence identity and gene sequence identity can be calculated by a program, such as maximum matching or search homology of GENETYX (manufactured by GENETYX), multiple alignment of CLUSTALW, or pairwise alignment of BLAST, herein. In order to calculate amino acid sequence identity, two or more luciferases may be aligned, and positions of identical amino acids in such two or more luciferases may be determined. The identical regions in amino acid sequences can be determined based on such information. The percent identity of two or more amino acid sequences is determined by subjecting two or more amino acid sequences to alignment using, for example, BLAST (TLASTP) targeting amino acid sequences by designating the total number of amino acids in the aligned region as the denominator and the number of identical amino acids relative to the total number as the numerator. If no identity is found in parts of the two or more amino acid sequences, for example, an amino acid sequence comprises at its C terminus an additional sequence in which no identity is observed, and, in general, such regions cannot be aligned and such regions are not used for calculation of the percent identity.

Further, positions of similar amino acids in two or more luciferases can be inspected. For example, a plurality of amino acid sequences can be subjected to alignment with the use of CLUSTALW. In such a case, Blosum62 is used as the algorithm and a plurality of amino acid sequences are subjected to alignment. Amino acids determined to be similar as a result of alignment may be referred to as "similar amino acids." In the mutant of the present disclosure, amino acid substitution can be carried out between such similar amino acids. Through such alignment, amino acid positions composed of the identical amino acids or similar amino acids among a plurality of amino acid sequences can be investigated. Based on such information, homologous regions (may be referred to as "highly conserved regions") in the amino acid sequences can be determined.

### (High-throughput screening)

A luciferase can further be subjected to high-throughput screening, so as to obtain a functional luciferase mutant. For example, a library of a transformant or transductant comprising the transgenic luciferase gene may be prepared and the resulting library may then be subjected to high-throughput screening using a microtiter plate. Alternatively, the library may be subjected to ultrahigh-throughput screening based on droplet microfluidics. For example, a combinatorial library of variant genes encoding variants can be constructed and a large population of variant luciferases can be subjected to screening by means of phage display (e.g., Chem. Rev., 105 (11): 4056-72, 2005), yeast display (e.g., Comb. Chem. High Throughput Screen., 2008; 11(2): 127-34), or bacterial display (e.g., Curr. Opin. Struct. Biol., 17: 474-80, 2007). Also see Agresti et al, "Ultrahigh-throughput screening in drop-based microfluidics for directed evolution," Proceedings of the National Academy of Sciences, 107 (9): 4004-4009, Mar, 2010. The contents of this document on the ultrahigh-throughput screening method that may be used for screening of a luciferase variant, is incorporated herein by reference. For example, a library can be constructed by error-prone PCR. Further, saturation mutagenesis may be used to introduce mutations into the region(s) or position(s) described herein or the corresponding region(s) or position(s) thereto as the target to construct a library. Using such library, appropriate cells such as electrocompetent EBY-100 cells, can be transformed and about 10 to the power of seven (10,000,000) mutants can be obtained. Yeast cells transformed with the library can then be subjected to cell sorting. A polydimethoxylsiloxane (PDMS) microfluidic device prepared via standard soft-lithography may be used. Monodisperse droplets can be formed using a flow-focusing device. Formed droplets containing individual mutants can be subjected to an appropriate sorting device. When screening cells, the presence or absence of luciferase activity can be utilized. For this purpose, a reaction solution having a composition capable of emitting light if luciferase reacts therewith, may, for example, be used. For example, a 96-well plate, a 192-well plate, a 384-well plate, or a 9600-well plate and a plate reader may be used to measure luminescence. Mutagenesis and screening may be repeated a plurality of times. The term mutation used here includes substitution, insertion, deletion, and/or addition of amino acid(s).

For example, 1 to 10 mutations can be introduced into a luciferase, and luciferase activity can be confirmed. Next, a luciferase mutant confirmed to have activity can be used as the starting material, further 1 to 10 mutations can be introduced thereinto and activity may be confirmed. A series of high throughput screening procedures (for example, the above method in which about 10 to the power of seven mutants are obtained and screened) may be repeated by 2 or more rounds, 5 or more rounds, 10 or more rounds, 15 or more rounds, for example, 20 or more rounds. High throughput screening to introduce 1 or more mutations, 5 or more mutations, for example, 10 or more mutations at each round may be repeated, for example, by 10 rounds to rapidly obtain mutants having 10 or more mutations, 50 or more mutations, for example, 100 or more mutations introduced in the starting luciferase and also having activity. Such high throughput screening may be repeated by 20 rounds to rapidly obtain mutants having 20 or more mutations, 100 or more mutations, for example, 200 or more mutations introduced into the starting luciferase and also having activity. These procedures may be performed by using an automatic apparatus or by repeating a routine process.

Mutation(s) may be introduced into any one or more positions from the first amino acid to the last amino acid of the full length amino acid sequence of the luciferase. However, regions important for enzyme functions, such as the active center, substrate recognition sites, ATP recognition sites and their neighborhoods are excluded. Luciferases are used in extensive in the industry, and those skilled in the art have a thorough knowledge on the regions important for enzyme functions, including an active center, substrate recognition site and the ATP recognition site of luciferases. In a particular embodiment, for example, one or more mutations may first be introduced into the part from the 1st to 10th positions of the full length sequence of the luciferase. Next, an luciferase mutant confirmed to have activity can be used as the starting material, and then one or more mutations can be introduced into the 11th to 20th positions and activity may be confirmed. This procedure may be repeated "n" times (n ≤ 55). For example, at the 55th round, one or more mutations can be introduced into positions 541 to 548. Regions important for enzyme functions or regions not intended to undergo modification may appropriately be skipped during the process. For example, position 208 of SEQ ID NO: 1 can be skipped. With this, it is possible to introduce a mutation into any position in the full length sequence except for the regions important for enzyme functions and luciferase mutants having, for example, 5 or more, 10 or more, 20 or more, 30 or more, 40 or more, 50 or more, 60 or more, 70 or more, 80 or more, 90 or more, 100 or more, 110 or more, 120 or more, 130 or more, 140 or more, 150 or more, 160 or more, 170 or more, 180 or more, 190 or more, for example, 200 or more mutations and having activity may be obtained rapidly. In an embodiment, a mutation that would eliminate luciferase activity is not introduced. In an embodiment, mutations are not introduced into positions corresponding to positions 202, 208, 346, 424, 439, 448, and 457 of SEQ ID NO: 1.

A mutation may be introduced randomly or may be introduced by rational design. In one embodiment, the mutation to be introduced by rational design or the mutation to be introduced at random may be a conservative amino acid substitution. Conservative amino acid substitutions include an amino acid substitution by which the amino acid before the substitution and the amino acid after the substitution have similar chemical characteristics (for example, Stryer et al., Biochemistry, Vol. 5, 2002, pp. 44 to 49). The conservative amino acid substitution may be selected from the group consisting of, for example, (i) a substitution of a basic amino acid with a different type of basic amino acid; (ii) a substitution of an acidic amino acid with a different type of acidic amino acid; (iii) a substitution of an aromatic amino acid with a different type of aromatic amino acid; (iv) a substitution of a nonpolar aliphatic amino acid with a different type of nonpolar aliphatic amino acid; and (v) a substitution of a polar uncharged amino acid with a different type of polar uncharged amino acid. The basic amino acid may be selected from, for example, arginine, histidine, and lysine. The acidic amino acid may be, for example, aspartic acid or glutamic acid. The aromatic amino acid may be selected from, for example, phenylalanine, tyrosine and tryptophan. The nonpolar aliphatic amino acid may be selected from, for example, glycine, alanine, valine, leucine, methionine, proline and isoleucine. The polar uncharged amino acid may be selected from, for example, serine, threonine, cysteine, asparagine and glutamine. With regard to the conservative amino acid substitution, the amino acid residue before the substitution and the amino acid residue after the substitution have similar chemical characteristics and positions at which the conservative amino acid substitution is performed are considered to exist at the same positions in the three-dimensional structures of protein and, therefore, it is highly plausible that variants having the conservative amino acid substitution maintain the three-dimensional structures and have activity.

In one embodiment, the mutation to be introduced by rational design or the mutation to be introduced at random includes a substitution between functionally similar amino acids. The list of functionally similar amino acids is well known in the art. In one embodiment, with regard to the substitution between functionally similar amino acids, the amino acid before the substitution and the amino acid after the substitution may fall into any category of amino acid classification defined as follows:
1) glycine (G), alanine (A);
2) aspartic acid (D), glutamic acid (E);
3) asparagine (N), glutamine (Q);
4) arginine (R), lysine (K), histidine (H);
5) isoleucine (I), leucine (L), valine (V), proline (P);
6) phenylalanine (F), tyrosine (Y), tryptophan (W);
7) serine (S), threonine (T); and
8) cysteine (C), methionine (M).

In contrast to the conservative amino acid substitution, a nonconservative amino acid substitution is any amino acid substitution that does not correspond to the conservative substitutions (i) to (v) summarized above, for an amino acid. In one embodiment, the amino acid substitution may be a nonconservative amino acid substitution. In this case, for example, whether luciferase activity is maintained before and after introduction of the nonconservative amino acid substitution may be confirmed. If the activity is confirmed, the nonconservative amino acid substitution may be adopted.

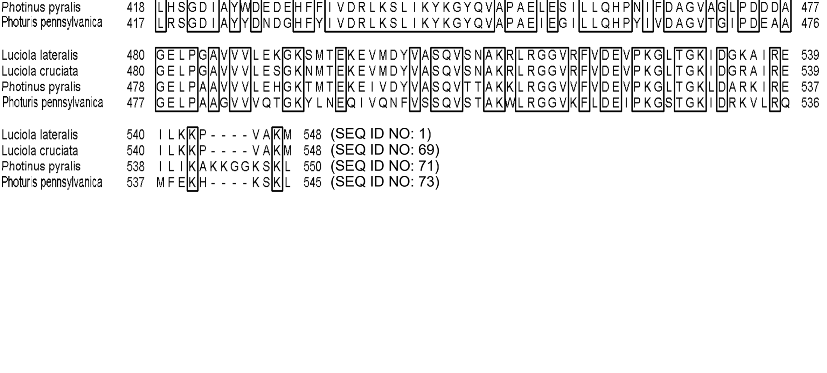

In an embodiment, amino acid substitution can be substitution of an amino acid with a similar amino acid (similarity substitution). As used herein, the substitution of an amino acid with a similar amino acid is substitution of the amino acid with an amino acid that is evaluated to have positive or neutral (zero) values in the amino acid substitution matrix used in the ClustalW software and the Blosum62, unless specified otherwise (e.g., S. Heinkoff and J. G. Henikoff, Proc. Natl. Acad. Sci., U.S.A., Vol. 89, pp. 10915-10919, 1992, see, in particular, Figure 2 and Thompson, Nucleic Acid Research, 1994, Vol. 22, No. 22, pp. 4673-4680). The matrix list was generated from aligned sequence segments of about 2000 blocks of 500 or more related proteins. Iterative application leads to nearly the same set of scores, even starting with a unitary matrix or using a subset of the protein group. Therefore, it is believed that these substitution matrices are versatile. This is an approach used most widely, which exploits the fact that sequences having a homology are evolutionarily related. Therefore, it is highly plausible that variants having a similarity substitution introduced in a given luciferase have activity. For example, in these substitution matrices, a substitution of serine with threonine is evaluated as the positive value "1" and thus, for example, a substitution of serine at position 24 of SEQ ID NO: 1 with threonine corresponds to a substitution with a similar amino acid. The position indicated above is threonine in SEQ ID NO: 71, and both enzymes have activity. Therefore, it is highly plausible that a S24T mutant of SEQ ID NO: 1 has activity. The same applies to variants subjected to other similarity substitutions.

In one embodiment, the conservative amino acid substitution or substitution an amino acid with a functionally similar amino acid does not reside in regions important for enzyme functions of the enzyme, such as the active center, substrate recognition sites, coenzyme recognition motifs and their neighborhoods and thus does not significantly influence the activity of the enzyme. In another embodiment, while the conservative amino acid substitution or substitution between functionally similar amino acids resides in the active center, substrate recognition sites, coenzyme recognition motifs and their neighborhoods, and the like in the enzyme, this does not substantially influence the activity of the enzyme.

The number of amino acids subjected to substitution, deletion, or addition indicated by the term "one or several" can be 1 to 10, for example, 1 to 9, 1 to 8, 1 to 7, 1 to 6, 1 to 5, 1 to 4, or 1 to 3, such as 1 or 2.

A luciferase mutant may comprise a deletion of an amino acid from the sequence before amino acid substitution. In a typical embodiment, the amino acid deletion does not reside in regions important for enzyme functions and, therefore, does not significantly influence the activity of the enzyme. In one embodiment, the deletion may be a short deletion of 1 to 2 amino acids. In one embodiment, when a given luciferase has an amino acid sequence that is different from the amino acid sequence of another luciferase by amino acid deletion, this deletion can be introduced into the other luciferase. Since both luciferases exhibit activity, such a deletion is likely to have no significant impact on the activity of the enzyme.

An example of amino acid deletion is provided. When SEQ ID NO: 73 is aligned with SEQ ID NO: 1, for example, the position corresponding to position 7 of SEQ ID NO: 1 is deleted in SEQ ID NO: 73. As such, if position 7 is deleted from SEQ ID NO: 1, it is highly likely that such deletion will not significantly influence the enzyme activity. Other deletions can be identified based on Figure 1.

A luciferase mutant may comprise an insertion of additional amino acids compared to the sequence before amino acid substitution. In a typical embodiment, the amino acid insertion does not reside in regions important for enzyme functions, such as the active center, substrate recognition sites, coenzyme recognition motifs and their neighborhoods and thus does not significantly influence the activity of the enzyme. In an embodiment, an "insertion" can be an insertion of 1 to 4 amino acids. When a given luciferase has an amino acid sequence that is different from the amino acid sequence of another luciferase by amino acid insertion, in one embodiment, such amino acid insertion can be introduced into the other luciferase. Since both luciferases exhibit activity, it is highly likely that such deletion will not significantly influence the enzyme activity.

An example of amino acid insertion is provided. When SEQ ID NO: 1 is aligned with SEQ ID NO: 73, proline is inserted into a position between position 39 and position 40 of SEQ ID NO: 1 and at the position corresponding to position 37 of SEQ ID NO: 73. Since both luciferases exhibit activity, it is highly likely that such insertion will not significantly influence the enzyme activity. As such, it is possible to insert an amino acid (e.g., proline) into the position between position 39 and position 40 of SEQ ID NO: 1. Other sites of insertion can be identified based on Figure 1. Amino acids to be inserted may be amino acids that are inserted into other sequence or amino acids that are conservative amino acid substitutions with such amino acids.

In addition, a luciferase mutant may comprise an addition of further amino acids compared to the sequence before amino acid addition. In a typical embodiment, amino acid addition is performed at the N or C terminus of a luciferase, and does not significantly influence the enzyme activity. In one embodiment, "addition" can be an addition of 1 to 6 amino acids or 1 to 5 amino acids, such as 1 to 4 amino acids. An example of addition may be, but is not limited to, a short stretch of histidine residues (e.g., 2 to 6 histidine residues) to assist luciferase purification. Another example of addition is, but is not limited to, addition of a signal peptide to assist luciferase expression. Examples of signal peptides include known signal sequences and sequences functionally equivalent thereto.

An example of amino acid addition is provided. When SEQ ID NO: 1 is aligned with SEQ ID NO: 71, in SEQ ID NO: 1, three amino acids are added to the region upstream of the N terminus of SEQ ID NO: 71. Since both luciferases exhibit activity, it is highly likely that such addition will not significantly influence the enzyme activity. Accordingly, it is possible to add 1, 2, or 3 amino acids to the N terminus of SEQ ID NO: 71. Amino acids to be added may be corresponding amino acids in the other sequence, or amino acids that are conservative amino acid substitutions with such amino acids.

A mutation can be introduced into a luciferase so as not to destroy a secondary structure or structural motif, such as an α helix structure or β sheet structure. A region of the secondary structure may be identified with, for example, a secondary structure predicting algorithm. Examples of the predicting algorithm include, but are not limited to, NetSurfP - 2.0. The same applies to other structural motifs such as nest or niche.

In one embodiment, an amino acid residue or an amino acid sequence motif that is essential for the activity of luciferase is not substituted. In another embodiment, even though a conservative amino acid substitution or similarity substitution can be performed at these positions, activity is to be confirmed with regard to a variant after the substitution. In one embodiment, amino acid deletion or insertion is not performed at a position downstream or upstream of an amino acid residue that is essential for luciferase activity. A position downstream or upstream of an amino acid residue that is essential for luciferase activity is a position closer to the N or C terminus by 1 or 2 positions from the amino acid residue that is essential for luciferase activity. In another embodiment, while amino acid deletion or insertion can be performed at a position downstream or upstream of an amino acid residue that is essential for luciferase activity, activity of a variant after substitution is to be confirmed. Whether or not a variant has activity can be confirmed by a routine process, such as high-throughput screening.

In an embodiment, the luciferase mutant of the present disclosure has luciferase activity. The presence or absence of the luciferase activity can be measured, for example, in accordance with the method described in Examples using Lumitester C-110 (manufactured by Kikkoman Biochemifa Company).

In the present description, "thermostability" can be evaluated by using, for example, the residual activity when a heat treatment is performed on the firefly luciferase at a predetermined temperature for a predetermined period of time as an indicator. Specifically, the thermostability of the firefly luciferase can be evaluated by comparing residual activity rates of the firefly luciferases after heat treatment under high temperature conditions, for example, at a reaction temperature usually from 30°C to 50°C, for example, from 35°C to 45°C or from 35°C to 40°C for a certain period of time, usually from 5 to 180 minutes or from 10 to 180 minutes, for example, from 60 to 180 minutes or about 90 minutes.

The residual activity of the firefly luciferase is activity after the heat treatment relative to the firefly luciferase activity before the action under the above-described high temperature condition, which is designated as 1. When the residual activity after the heat treatment is halved, for example, the residual activity after the heat treatment is 0.5 relative to the activity before the heat treatment, which is designated as 1. The residual activity percentage of the firefly luciferase after the heat treatment is calculated as the ratio relative to the firefly luciferase activity before the action under the above-described high temperature condition. The term "improved thermostability" used herein refers to the residual activity percentage, when the firefly luciferase mutant is subjected to the reaction under the above-identified conditions, being improved by at least 1.01 times, 1.02 times, 1.1 times, 1.2 times, 1.3 times, 1.4 times, 1.5 times, 1.6 times, 1.7 times, 1.8 times, 1.9 times, or 2 times, relative to the residual activity of the luciferase before introduction of the mutations of the present disclosure (i.e., mutations at positions corresponding to position 252 and the like of SEQ ID NO: 1). In other words, the term "improved thermostability" used herein refers to the residual activity percentage, when the firefly luciferase mutant is subjected to the reaction under the above-identified conditions, being improved by at least 1%, 2%, 10%, 20%, 30%, 40%, 50%, 60%, 70%, 80%, 90%, or 100%, relative to the residual activity percentage of the luciferase before introduction of the mutations of the present disclosure.

### (Polynucleotide)

In an embodiment, the present disclosure provides a polynucleotide encoding the luciferase mutant (hereinafter also referred to as a "luciferase gene"). A sequence of a polynucleotide can readily be determined based on the amino acid sequence of the firefly luciferase mutant. For example, the polynucleotide encoding the amino acid sequence of SEQ ID NO: 2 or 10 include the polynucleotide of SEQ ID NOs: 3 or 11, respectively. For example, the polynucleotide encoding the luciferase before amino acid substitution may comprise:
(i) a nucleotide sequence exhibiting 85% or higher, 86% or higher, 87% or higher, 88% or higher, 89% or higher, and more preferably 90% or higher, 91% or higher, 92% or higher, 93% or higher, 94% or higher, 95% or higher, 96% or higher, 97% or higher, 98% or higher, or 99% or higher sequence identity to the nucleotide sequence of SEQ ID NO: 3 or 11 over the entire length and encoding an active luciferase;
(ii) a nucleotide sequence derived from the nucleotide sequence of SEQ ID NO: 3 or 11 by substitution, deletion, or addition of one or several nucleotides and encoding an active luciferase; or
(iii) a nucleotide sequence selected from the group consisting of the nucleotide sequences of SEQ ID NOs: 3 and 11.

In order to obtain nucleotides encoding these luciferases, methods for cloning genes used in general can be applied. For example, chromosomal DNA or mRNA can be extracted from tissues or cells of a firefly having a luciferase producing ability by conventional methods, for example, the method described in Current Protocols in Molecular Biology (WILEY Interscience, 1989). Further, cDNA can be synthesized using the mRNA as the template. The chromosomal DNA or cDNA thus obtained can be used to produce a library of the chromosomal DNA or the cDNA.

Next, an appropriate probe DNA can be synthesized based on the amino acid sequence of the luciferase above and a luciferase LOD gene can be screened from the chromosomal DNA or cDNA library by using the probe DNA or, alternatively, appropriate primer DNA(s) can be prepared based on the amino acid sequence above and the DNA containing the gene fragment of interest encoding the luciferase can be amplified with an appropriate polymerase chain reaction (PCR method) such as the 5'RACE method and the 3'RACE method, and then, these DNA fragments can be linked to obtain a DNA containing the full-length luciferase gene of interest.

When the nucleotide sequence encoding the luciferase is already-known, the nucleotide sequence may be artificially synthesized. Artificial gene synthesizing service is provided by, for example, Integrated DNA Technologies.

### (Production method of luciferase gene)

The mutation treatment of the luciferase gene can be performed by any known method according to the intended mutation form. That is, a method that brings the luciferase gene or recombinant DNA, into which this gene is incorporated, into contact with an agent serving as a mutagen so as to allow the agent to act on the gene or DNA; an ultraviolet irradiation method; a genetic engineering method; a method making full use of protein engineering methods, or the like can be widely used.

Examples of the agent serving as the mutagen used for the mutation treatment above include hydroxylamine, N-methyl-N'-nitro-N-nitrosoguanidine, nitrous acid, sulfurous acid, hydrazine, formic acid, and 5-bromouracil.

Regarding various conditions of the contact and the action, conditions depending on the type of agent to be used or the like can be employed, and the conditions are not specifically limited as long as a desired mutation can be actually induced in the luciferase gene. Usually, the contact and the action with the agent at a concentration of preferably 0.5 to 12 M, a reaction temperature of 20°C to 80°C for 10 minutes or more and preferably from 10 to 180 minutes allows for inducing the desired mutation. When ultraviolet irradiation is performed, it can be carried out in accordance with conventional methods as described above (Modern Chemistry, 024 to 30, June 1989 issue).

As a method making full use of protein engineering methods, in general, a method known as site-directed mutagenesis can be used. Examples include the Kramer method (Nucleic Acids Res., 12, 9441-9456, 1984), the Eckstein method (Nucleic Acids Res., 13, 8749-8764, 1985: Nucleic Acids Res., 13, 8765, 1985: Nucleic Acids Res, 14, 9679, 1986), and the Kunkel method (Proc. Natl. Acid. Sci. U.S.A., 82, 488-492, 1985).

In addition to the genetic modification method above, a desired modified luciferase gene can be directly synthesized using an organic synthesis method or an enzyme synthesis method.

The nucleotide sequence of the luciferase gene can be confirmed by, for example, a multi-capillary DNA analysis system, Applied Biosystems 3730xl DNA analyzer (manufactured by Thermo Fisher Scientific).

### (Vector, host cell)

In an embodiment, the present disclosure relates to a vector comprising the polynucleotide. Preferably, these luciferase genes are ligated to various types of vectors in accordance with conventional methods for ease of handling. While an example of a vector includes a plasmid, any other vectors known to a person skilled in the art, such as a bacteriophage vector and a cosmid vector, can be used. The type of the vector can be selected depending on the host cell, and, specifically, pET16-b, pKK223-3, or the like can preferably be used.

In one embodiment, the present disclosure relates to a host cell comprising the polynucleotide or vector. The host cell is not limited, it is a bacterium, such as Escherichia coli or Bacillus subtilis, a yeast cell, an insect cell, an animal cell (for example, a mammal cells), or a plant cell, and it is preferably a bacterial cell, such as Escherichia coli.

### (Transformation and transduction)

The luciferase gene obtained as described above can be incorporated into a vector, such as a bacteriophage, cosmid, or plasmid vector, used for transformation of a prokaryotic cell or a eukaryotic cell, by conventional methods, and the transformation or transduction can be performed on the host corresponding to each vector by conventional methods. For example, a microorganism belonging to the genus Escherichia may be used as the host, the obtained recombinant DNA may be used, and transformation or transduction may be performed, for example, on the Escherichia coli K-12 strain or the Escherichia coli B strain, and preferably the Escherichia coli JM109 strain, the Escherichia coli DH5α strain, the Escherichia coli BL21 strain, the Escherichia coli BL21 (DE3) strain (all of them are manufactured by Takara Bio), and the like to obtain relevant strains. As a method for introducing a recombinant vector into a host cell, if the host cell is a microorganism belonging to Escherichia coli, a method of transferring recombinant DNA in the presence of a calcium ion can be employed. Furthermore, an electroporation method may be used. Moreover, commercially available competent cells (for example, ECOS Competent Escherichia coli BL21 (DE3); manufactured by Nippon Gene Co., Ltd.) may be used. The luciferase gene may be a codon-optimized gene depending on the expression host.

### (Production method of a luciferase mutant)

In an embodiment, the present disclosure relates to the production method of the luciferase mutant having improved thermostability comprising a step of culturing the host cell. Culture can be performed by various kinds of known methods, and a method of solid culture can be employed, with a method of liquid culture being preferable.

The production method may comprise a step of culturing the host cell under a condition in which a luciferase protein can be expressed and optionally a step of isolating the luciferase from the culture product or culture fluid. Conditions under which the luciferase protein can be expressed refer to conditions under which a luciferase gene is transcribed and translated, and a polypeptide encoded by such gene is produced.

In an embodiment, the production method comprises artificially introducing a mutation at the position corresponding to position 252 and the like of SEQ ID NO: 1 in a luciferase protein before the step of culture. This can be performed by artificially introducing the mutation to the sequence of a gene encoding the luciferase.

As the medium for culturing the host cell, for example, a medium produced by adding one or more kinds of inorganic salts, such as sodium chloride, potassium dihydrogen phosphate, dipotassium hydrogenphosphate, magnesium sulfate, magnesium chloride, ferric chloride, ferric sulfate, or manganese sulfate, to one or more kinds of nitrogen sources, such as a yeast extract, triptone, peptone, a meat extract, corn steep liquor, or exudate of soybean or wheat bran, and further adding a carbohydrate raw material, vitamin, or the like as necessary is used.

The initial pH of the medium is appropriately adjusted to 7 to 9. Culture is performed at a cultivation temperature of 20°C to 42°C, preferably at a cultivation temperature of around 25°C to 37°C for 4 to 24 hours, and further preferably at a cultivation temperature of around 25°C to 37°C for 8 to 16 hours by aeration-agitation submerged culture, shake culture, static culture, or the like.

After the completion of culture, the luciferase can be collected from the cultured product by a conventional means of collecting enzymes. For example, the enzyme of interest can be released from a microorganism body by performing ultrasonic disruption treatment, milling treatment, or the like on the microorganism body by conventional methods, extracting the present enzyme with lytic enzymes, such as lysozyme, or shaking or allowing to stand still in the presence of toluene or the like for lysis. This solution is, for example, filtrated or centrifuged to remove solid matter, nucleic acids are removed with streptomycin sulfate, protamine sulfate, manganese sulfate, or the like, according to need, ammonium sulfate, alcohol, acetone, or the like is added thereto, fractionation is performed, a precipitate is collected, and a crude enzyme of the luciferase is then obtained.

In order to further obtain a purified luciferase enzyme from the crude enzyme of the luciferase, for example, a gel filtration method using Sephadex, Superdex, Ultro-gel, or the like, an adsorption elution method using an ion exchange carrier, a hydrophobic carrier, or hydroxyapatite, an electrophoresis method using polyacrylamide gel or the like, a sedimentation method, such as a sucrose density-gradient centrifugation, an affinity chromatography method, or a fractionation method using a molecular sieving membrane, a hollow fiber membrane, or the like is appropriately selected and performed, or a combination of the same is performed, to obtain the purified luciferase enzyme. The desired luciferase can be thus obtained.

The produced luciferase can be used in the kit described herein or in a method for detecting at least one of ATP, ADP, and AMP.

### (Kit for detecting at least one of ATP, ADP, and AMP)

In an embodiment, the present disclosure relates to a kit for detecting at least one of ATP, ADP, and AMP, which comprises the luciferase mutant described herein. The kit may comprise luciferin in addition to the luciferase mutant. In this case, metal ions, such as magnesium, manganese, and calcium ions, can also be included in the kit. Those skilled in the art can determine the concentration of the metal ions depending on the enzyme to be used. Luciferase converts ATP, O₂, and luciferin into AMP, pyrophosphoric acid, CO₂, and oxyluciferin, and, during the process of conversion, luminescence is induced. The reaction that occurs during the process of conversion is expressed as follows.

Luciferin + ATP + O₂ → oxyluciferin + adenosine monophosphate (AMP) + pyrophosphoric acid (PPi) + CO₂ + light

In an embodiment, the kit further comprises an enzyme catalyzing a reaction of generating ATP from ADP. This enzyme catalyzing the reaction of generating ATP from ADP can be selected from the group consisting of pyruvate kinase (PK), acetate kinase (AK), creatine kinase (CK), polyphosphate kinase (PPK), hexokinase, glucokinase, glycerol kinase, fructokinase, phosphofructokinase, riboflavin kinase, and fructose-bisphosphatase. In another embodiment, the kit further comprises pyruvate orthophosphate dikinase (PPDK), adenylate kinase (ADK), or pyruvate-water dikinase (PWDK).

If ATP is contained in a sample, it is converted into AMP by the luciferase, and luminescence is also generated. If ADP is contained in a sample in a system where an enzyme catalyzing the reaction of generating ATP from ADP is present, the enzyme converts ADP into ATP, and ATP is then subjected to a luminescence reaction. This enables measurement of the total quantity of ATP and ADP present in the system. If AMP is contained in a sample in a system where PPDK is present, AMP is converted into ATP by PPDK, PEP, and PPi. If AMP is contained in a sample in a system where PWDK is present, AMP is converted into ATP by PWDK, PEP, and phosphoric acid. The generated ATP produces luminescence again by the luciferase. Since the luminescence is stably maintained and the amount of luminescence is correlated with the total quantity of ATP and AMP in the system, ATP and AMP can be quantitated. The presence of an enzyme catalyzing the reaction of generating ATP from ADP and PPDK, ADK, or PWDK enables measurement of the total quantity of ATP, ADP, and AMP.

The luciferin may be any luciferin as long as it is recognized as a substrate by the luciferase to be used and may be natural or chemically synthesized. Moreover, any known luciferin derivative can also be used. The basic structure of luciferin is imidazopyrazinone, and there are many tautomers thereof. Luciferin includes firefly luciferin. The firefly luciferin is a substrate of firefly luciferase (EC 1.13.12.7). The luciferin derivative can be those described in JP 2007-91695 A and JP 2010-523149 T (WO 2008/127677) and the like.

In addition to the components above, the kit may include at least one of a stabilizing agent, a buffer, and instructions for use.

### (Method for detecting at least one of ATP, ADP, and AMP)

In an embodiment, the present disclosure relates to a method that detects at least one of ATP, ADP, and AMP, comprising using the luciferase mutant described herein. This method may comprise a step of catalyzing an oxidation reaction of the luciferin using the luciferase mutant described herein and a step of measuring luminescence generated by the oxidation reaction.

The catalyst of the oxidation reaction of the luciferin by the luciferase mutant is as described in "Kit for detecting at least one of ATP, ADP, or AMP." The reaction can be performed by allowing the luciferase mutant and the luciferin described herein to react with a sample. If ATP is contained in the sample, ATP is converted into AMP by the luciferase, and luminescence is generated. This enables measurement of ATP. In a system where an enzyme catalyzing the reaction of generating ATP from ADP is present, the total quantity of ATP and ADP present in the system can be measured. In a system where PPDK or PWDK is present, in addition, ATP and AMP can be quantitated. If an enzyme catalyzing the reaction of generating ATP from ADP and PPDK, ADK, or PWDK is present, the total quantity of ATP, ADP, and AMP can be measured.

The amount of luminescence from the luciferase can be measured by known methods and can be evaluated using relative luminescence intensity (RLU) as the indicator which is obtained using, for example, an appropriate apparatus for measuring luminescence, such as a luminometer (CentroLB960 or Lumat3 LB9508 manufactured by Berthold Technologies GmbH & Co. KG; Lumitester C-110, Lumitester C-100, Lumitester PD-20, or Lumitester PD-30 manufactured by Kikkoman Biochemifa Company, or the like). Typically, luminescence generated during conversion of luciferin to oxyluciferin is measured. As the apparatuses for measuring luminescence, apparatuses capable of high sensitivity measurement and equipped with a photomultiplier tube (those manufactured by 3M Corporation and the like) and apparatuses equipped with a photodiode (those manufactured by Hygiena, LLC, Neogen Corporation, and the like) can also be used.

In one embodiment, with regard to the luciferase mutant of the present disclosure (e.g., a luciferase mutant having an amino acid sequence exhibiting at least 70%, 85%, 80%, 85%, 90%, or 95% identity to SEQ ID NO: 10 and in which position corresponding to position 252 of SEQ ID NO: 1 is leucine), the position corresponding to position 393 of SEQ ID NO: 1 is isoleucine, position corresponding to position 326 of SEQ ID NO: 1 is serine, position corresponding to position 392 of SEQ ID NO: 1 is isoleucine, and position corresponding to position 467 of SEQ ID NO: 1 is isoleucine. In one embodiment, such mutant has at least one amino acid substitution corresponding to 252L, 262Y, 294L, 90Y, 371L, 41V, 427F, 53L, 306F, 221L, 17D, 17E, 17K, 18L, 75K, 110D, 110E, 110K, 110R, 223V, 223L, 223I, 224I, 257F, 229F, 229L, 158D, or 158E when taking SEQ ID NO: 1 as the reference sequence.

Wild-type sequences are excluded from the scope of the luciferase mutant of the present disclosure. Naturally occurring products are also excluded from the scope of the luciferase mutant of the present disclosure.

In an embodiment, the peroxisome targeting signal (PTS) 1 can be deleted from a luciferase. PTS1 is the 3 amino acids at the C terminus, and the consensus sequence thereof is "-(S/A/C)-(K/R/H)-(L/M)-COOH." In an embodiment, for example, 3, 4, or 5 amino acids can be deleted from the C terminus of the luciferase although the present disclosure is not limited thereto. In an embodiment, for example, 6, 7, 8, 9, 10, 11, or 12 amino acids can be deleted from the C terminus of the luciferase although the present disclosure is not limited thereto. Whether or not a deletion mutant has activity can be readily confirmed by a routine process.

Hereafter, the present invention is described in greater detail with reference to the examples. It should be noted that the technical scope of the present invention is not limited to these examples in any way.

### [Examples]

The mutant luciferase of the present disclosure is described in detail with reference to the following examples. It should be noted that these examples are provided merely for illustration and the present disclosure is not limited to these examples in any way.

pET-16b-LlLuc-1M is a plasmid for expressing luciferase, which is prepared by inserting a gene (SEQ ID NO: 3) encoding a mutant Luciola lateralis luciferase (SEQ ID NO: 2) into the multicloning site of the pET-16b vector (see WO 2020/009215).

### 1. Preparation of luciferase mutants from Luciola lateralis (LlLuc)

Site-directed mutagenesis was performed by PCR using pET-16b-LlLuc-1M as a template. The reagent composition and the reaction conditions for PCR were in accordance with the instructions of KOD One PCR Master Mix (TOYOBO). The reaction was performed by repeating a cycle of "98°C for 10 seconds, 55°C for 5 seconds, and 68°C for 30 seconds" 15 times.

DpnI (1 µl) was added to 20 µl of the PCR product, and the resultant was allowed to react at 37°C for 1 hour to decompose the template DNA. Subsequently, E. coli JM109 was transformed using the reaction product. The obtained transformant was cultured in LB medium containing 50 µg/ml ampicillin, cells were collected by centrifugation, and a plasmid retaining a gene encoding the LlLuc-1M mutant was extracted from the transformant using the FastGene Plasmid Mini Kit (Nippon Genetics Co., Ltd.). A multiple mutant LlLuc-expressing plasmid was constructed by repeating introduction of single mutations. For example, PCR was carried out using pET-16b-LlLuc-1M as the template and primers shown in SEQ ID NO: 4 and SEQ ID NO: 5 to construct the LlLuc-2M-expressing plasmid. The names of the mutants prepared, the amino acid substitutions introduced into LlLuc-1M, and the template plasmids and the primers used in PCR are shown in Table 1.

**[Table 1]**

| Name of mutant | Amino acid substitution introduced into LlLuc-1M | Template | Rv primer | Fw primer |
|---|---|---|---|---|
| LlLuc-2M | G326S | pET-16b-LlLuc-1M | SEQ ID NO: 4 | SEQ ID NO: 5 |
| LlLuc-3M | G326S, V392I | pET-16b-LlLuc-2M | SEQ ID NO: 6 | SEQ ID NO: 7 |
| LlLuc-4M | G326S, V392I, F467I | pET-16b-LlLuc-3M | SEQ ID NO: 8 | SEQ ID NO: 9 |

### 2. Preparation of LlLuc-4M mutant

In accordance with the method described in the "1. Preparation of luciferase mutants from Luciola lateralis (LlLuc)" section above, mutants of LlLuc-4M (SEQ ID NO: 10) were prepared. The nucleotide sequence of the gene encoding LlLuc-4M is shown in SEQ ID NO: 11. Multiple mutated LlLuc-4M-expressing plasmids were constructed by repeating introduction of single mutations. For example, PCR was carried out using pET-16b-LlLuc-5M as the template and primers shown in SEQ ID NO: 16 and SEQ ID NO: 17 to construct the LlLuc-6M-expressing plasmid. The names of the mutants prepared, the amino acid substitutions introduced into LlLuc-4M, and the template plasmids and the primers used in PCR are shown in Table 2.

**[Table 2]**

| Name of mutant | Amino acid substitution introduced into LlLuc-4M | Template | Rv primer | Fw primer |
|---|---|---|---|---|
| LlLuc-4M-1 | F90Y | pET-16b-LlLuc-4M | SEQ ID NO: 12 | SEQ ID NO: 13 |
| LlLuc-4M-2 | F221L | pET-16b-LlLuc-4M | SEQ ID NO: 14 | SEQ ID NO: 15 |
| LlLuc-4M-3 | F252L | pET-16b-LlLuc-4M | SEQ ID NO: 16 | SEQ ID NO: 17 |
| LlLuc-4M-4 | F262Y | pET-16b-LlLuc-4M | SEQ ID NO: 18 | SEQ ID NO: 19 |
| LlLuc-4M-5 | F371L | pET-16b-LlLuc-4M | SEQ ID NO: 20 | SEQ ID NO: 21 |
| LlLuc-5M | F294L | pET-16b-LlLuc-4M | SEQ ID NO: 22 | SEQ ID NO: 23 |
| LlLuc-6M | F252L, F294L | pET-16b-LlLuc-5M | SEQ ID NO: 16 | SEQ ID NO: 17 |
| LlLuc-7M | F252L, F262Y, F294L | pET-16b-LlLuc-6M | SEQ ID NO: 24 | SEQ ID NO: 19 |

### 3. Recombinant production of LlLuc-4M mutant

The LlLuc-4M- or mutant LlLuc-4M-expressing plasmid was used to transform E. coli BL21 (DE3), and LlLuc-4M- or mutant LlLuc-4M-producing strains were obtained. These strains were inoculated into 2 ml of LB medium containing 50 µg/ml ampicillin and 0.1 mM IPTG and then cultured at 25°C or 30°C and 180 rpm for 20 hours.

Pellets obtained by subjecting the culture solution to centrifugation at 15,000 rpm for 2 minutes were re-suspended in 1 ml of 230 mM tricine buffer (pH 7.85). The cell suspension was ultrasonically disintegrated, the resultant was centrifuged at 15,000 rpm for 15 minutes to obtain a supernatant, and the supernatant was collected as a crude enzyme solution of LlLuc-4M or mutant enzyme thereof.

### 4. Evaluation of thermostability of LlLuc-4M mutants

A crude enzyme solution (50 µl) of LlLuc-4M or mutant enzyme thereof was mixed with 50 µl of a reagent for heat treatment (19 mM ethylene-diamine-tetraacetic acid disodium salt, 0.4 mM dithiothreitol, 74 mM magnesium acetate, 20% xylitol, 0.2% Tween^{tm} 80), and the mixture was heated at 50°C for 120 minutes to evaluate the residual activity of the enzyme (Table 3).

**[Table 3]**

| Name of mutant | Amino acid substitution introduced into LlLuc-4M | Residual activity (50°C, 120 min) |
|---|---|---|
| LlLuc-4M | | 0.34 |
| LlLuc-4M-1 | F90Y | 0.87 |
| LlLuc-4M-2 | F221L | 0.87 |
| LlLuc-4M-3 | F252L | 0.96 |
| LlLuc-4M-4 | F262Y | 0.73 |
| LlLuc-4M-5 | F371L | 0.53 |
| LlLuc-5M | F294L | 0.66 |

The residual activities of the LlLuc-4M mutants shown in Table 3 were increased by 0.19 to 0.62, compared with that of LlLuc-4M. In other words, the residual activities of the LlLuc-4M mutants were enhanced by 58% to 187% and this indicates that all the LlLuc-4M mutants exhibited improved thermostability.

### 5. Recombinant production of LlLuc-4M, 5M, 6M, and 7M mutants

The LlLuc-4M-, 5M-, 6M-, and 7M-expressing plasmids were used to transform E. coli BL21 (DE3), and LlLuc-4M- or mutant LlLuc-4M-producing strains were obtained. These strains were inoculated into 2.5 ml of LB medium containing 100 µg/ml ampicillin and then precultured at 30°C and 160 rpm for 12 hours. A preculture solution (1 ml) was inoculated into 100 ml of 2× LB medium containing 50 µg/ml ampicillin and then cultured at 30°C and 120 rpm for 21 hours. Thereafter, 1 ml of 10 mM IPTG was added thereto and culture was performed at 28°C and 110 rpm for 17 hours.

Pellets obtained by subjecting the culture solution to centrifugation at 10,000 rpm for 10 minutes were suspended again in 40 ml of buffer A (5% glycerol, 1 mM EDTA-2Na, 2 mM 3-mercapto-1,2-propanediol, phosphoric acid, pH 7.1). The cell suspension was ultrasonically disintegrated, the resultant was centrifuged at 10,000 rpm for 50 minutes to obtain a supernatant, pH of the supernatant was adjusted to 7.1 with phosphoric acid, and crude enzyme solutions filtered through a 0.2-µm filter were collected.

The crude enzyme solution was applied to the cation-exchange chromatography column (HiScreen SP FF, Cytiva) equilibrated with the buffer A and subjected to gradient elution with buffer B (200 mM potassium phosphate, 5% glycerol, 1 mM EDTA-2Na, 2 mM 3-mercapto-1,2-propanediol, pH 7.1) and active fractions were collected. The active fractions were concentrated to approximately 30-fold with Amicon Ultra-3K (Merck) to obtain a purified enzyme solution.

### 6. Evaluation of thermostability of LlLuc-4M, 5M, 6M, and 7M mutants

The purified enzyme solution was diluted to at least 500-fold with a buffer for heat treatment (0.3M tricine, 0.2% BSA, 5% glycerol), and the resultant was heated at 60°C for 5 minutes to evaluate the residual activity of the enzymes (Table 4).

**[Table 4]**

| Name of mutant | Amino acid substitution introduced into LlLuc-4M | Residual activity (60°C, 5 min) |
|---|---|---|
| LlLuc-4M | | 0.13 |
| LlLuc-5M | F294L | 0.20 |
| LlLuc-6M | F252L, F294L | 0.54 |
| LlLuc-7M | F252L, F262Y, F294L | 0.65 |

The residual activities of the LlLuc-5M, 6M, and 7M mutants shown in Table 4 were increased by 0.07 to 0.52, compared with that of LlLuc-4M. In other words, the residual activities of the LlLuc-5M, 6M, and 7M mutants were enhanced by 54% to 400% and this indicates that thermostability of the LlLuc-4M mutants were enhanced drastically by combining the amino acid substitutions shown in Table 3.

### 7. Preparation of LlLuc-7M mutants

Site-directed mutagenesis was performed by PCR using pET-16b-LlLuc-7M as the template and primers shown in SEQ ID NOs: 25 and 26. The reagent composition and the reaction conditions for PCR were in accordance with the instructions of KOD One PCR Master Mix (TOYOBO). The reaction was performed by repeating a cycle of "98°C for 10 seconds, 55°C for 5 seconds, and 68°C for 30 seconds" 7 times.

DpnI (1 µl) was added to 20 µl of the PCR product, and the resultant was allowed to react at 37°C for 1 hour to decompose the template DNA. Subsequently, 5 µl of Ligation high Ver.2 (TOYOBO), 1 µl of T4 polynucleotide kinase (TOYOBO), and 7 µl of ion exchange water were added to 2 µl of the reaction product, the reaction was allowed to proceed at 16°C for 1 hour, and E. coli JM109 was then transformed using the reaction solution. The obtained transformant was cultured in LB medium containing 50 µg/ml ampicillin, cells were collected by centrifugation, and a plasmid retaining a gene (SEQ ID NO: 28) encoding the LlLuc-7Md (SEQ ID NO: 27) was extracted from the transformant using the FastGene Plasmid Mini Kit. LlLuc-7Md is a mutant prepared by deleting C-terminal 5 amino acids (P544, V545, A546, K547, and M548) of LlLuc-7M including the peroxisome targeting signal 1 (PTS1). PTS1 is the 3 amino acids at the C terminus, and the consensus sequence thereof is "-(S/A/C)-(K/R/H)-(L/M)-COOH" (see, for example, FEBS J., 272, 2362, 2005, Plant Cell Physiol., 38, 759, 1997, and Eur. J. Cell Biol., 71, 248, 1996).

In accordance with the method described in the "1. Preparation of luciferase mutants from Luciola lateralis (LlLuc)" section above, subsequently, mutants of LlLuc-7Md were prepared. Multiple mutated LlLuc-7Md-expressing plasmids were constructed by repeating introduction of single mutations. For example, PCR was carried out using pET-16b-LlLuc-8Md as the template and primers shown in SEQ ID NO: 66 and SEQ ID NO: 67 to construct the LlLuc-9Md-expressing plasmid. The names of the mutants prepared, the amino acid substitutions introduced into LlLuc-7Md, and the template plasmids and the primers used in PCR are shown in Table 5.

**[Table 5]**

| Name of mutant | Amino acid substitution introduced into LlLuc-7Md | Template | Rv primer | Fw primer |
|---|---|---|---|---|
| LlLuc-7Md-1 | F17D | pET-16b-LlLuc-7Md | SEQ ID NO: 29 | SEQ ID NO: 30 |
| LlLuc-7Md-2 | F17E | pET-16b-LlLuc-7Md | SEQ ID NO: 29 | SEQ ID NO: 31 |
| LlLuc-7Md-3 | F17K | pET-16b-LlLuc-7Md | SEQ ID NO: 29 | SEQ ID NO: 32 |
| LlLuc-7Md-4 | Y18L | pET-16b-LlLuc-7Md | SEQ ID NO: 33 | SEQ ID NO: 34 |
| LlLuc-7Md-5 | A41V | pET-16b-LlLuc-7Md | SEQ ID NO: 35 | SEQ ID NO: 36 |
| LlLuc-7Md-6 | Y53L | pET-16b-LlLuc-7Md | SEQ ID NO: 37 | SEQ ID NO: 38 |
| LlLuc-7Md-7 | I110D | pET-16b-LlLuc-7Md | SEQ ID NO: 39 | SEQ ID NO: 40 |
| LlLuc-7Md-8 | I110E | pET-16b-LlLuc-7Md | SEQ ID NO: 39 | SEQ ID NO: 41 |
| LlLuc-7Md-9 | I110K | pET-16b-LlLuc-7Md | SEQ ID NO: 39 | SEQ ID NO: 42 |
| LlLuc-7Md-10 | I110R | pET-16b-LlLuc-7Md | SEQ ID NO: 39 | SEQ ID NO: 43 |
| LlLuc-7Md-11 | V158D | pET-16b-LlLuc-7Md | SEQ ID NO: 44 | SEQ ID NO: 45 |
| LlLuc-7Md-12 | V158E | pET-16b-LlLuc-7Md | SEQ ID NO: 44 | SEQ ID NO: 46 |
| LlLuc-7Md-13 | H223V | pET-16b-LlLuc-7Md | SEQ ID NO: 47 | SEQ ID NO: 48 |
| LlLuc-7Md-14 | H223I | pET-16b-LlLuc-7Md | SEQ ID NO: 47 | SEQ ID NO: 49 |
| LlLuc-7Md-15 | H223L | pET-16b-LlLuc-7Md | SEQ ID NO: 47 | SEQ ID NO: 50 |
| LlLuc-7Md-16 | A224I | pET-16b-LlLuc-7Md | SEQ ID NO: 51 | SEQ ID NO: 52 |
| LlLuc-7Md-17 | Y229F | pET-16b-LlLuc-7Md | SEQ ID NO: 53 | SEQ ID NO: 54 |
| LlLuc-7Md-18 | Y229L | pET-16b-LlLuc-7Md | SEQ ID NO: 53 | SEQ ID NO: 55 |
| LlLuc-7Md-19 | Y257F | pET-16b-LlLuc-7Md | SEQ ID NO: 56 | SEQ ID NO: 57 |
| LlLuc-7Md-20 | Y306F | pET-16b-LlLuc-7Md | SEQ ID NO: 58 | SEQ ID NO: 59 |
| LlLuc-7Md-21 | V312R | pET-16b-LlLuc-7Md | SEQ ID NO: 60 | SEQ ID NO: 61 |
| LlLuc-7Md-22 | V312E | pET-16b-LlLuc-7Md | SEQ ID NO: 60 | SEQ ID NO: 62 |
| LlLuc-7Md-23 | V312K | pET-16b-LlLuc-7Md | SEQ ID NO: 60 | SEQ ID NO: 63 |
| LlLuc-7Md-24 | Y427F | pET-16b-LlLuc-7Md | SEQ ID NO: 64 | SEQ ID NO: 65 |
| LlLuc-8Md | V75K | pET-16b-LlLuc-7Md | SEQ ID NO: 66 | SEQ ID NO: 67 |
| LlLuc-9Md | V75K, I110D | pET-16b-LlLuc-8Md | SEQ ID NO: 39 | SEQ ID NO: 40 |
| LlLuc-10Md | V75K, I110D, V312K | pET-16b-LlLuc-9Md | SEQ ID NO: 60 | SEQ ID NO: 63 |
| LlLuc-11Md | F17E, V75K, I110D, V312K | pET-16b-LlLuc-10Md | SEQ ID NO: 29 | SEQ ID NO: 31 |
| LlLuc-12Md | F17E, V75K, I110D, V158D, V312K | pET-16b-LlLuc-11Md | SEQ ID NO: 44 | SEQ ID NO: 45 |
| LlLuc-13Md | F17E, A41V, V75K, I110D, V158D, V312K | pET-16b-LlLuc-12Md | SEQ ID NO: 35 | SEQ ID NO: 36 |
| LlLuc-14Md | F17E, A41V, V75K, I110D, V158D, Y229L, V312K | pET-16b-LlLuc-13Md | SEQ ID NO: 53 | SEQ ID NO: 55 |
| LlLuc-15Md | F17E, A41V, V75K, I110D, V158D, Y229L, Y257F, V312K | pET-16b-LlLuc-14Md | SEQ ID NO: 56 | SEQ ID NO: 57 |
| LlLuc-16Md | F17E, A41V, Y53L, V75K, I110D, V158D, Y229L, Y257F, V312K | pET-16b-LlLuc-15Md | SEQ ID NO: 37 | SEQ ID NO: 38 |
| LlLuc-15Md-2 | F17E, A41V, Y53L, V75K, I110D, V158D, Y257F, V312K | pET-16b-LlLuc-16Md | SEQ ID NO: 53 | SEQ ID NO: 68 |

### 8. Evaluation of thermostability of LlLuc-7Md mutants

In accordance with the method described in the "3. Recombinant production of LlLuc-4M mutant" section above, a crude enzyme solution of LlLuc-7Md or mutant LlLuc-7Md was prepared. In accordance with the method described in the "4. Evaluation of thermostability of LlLuc-4M mutants" section above, the residual activity of LlLuc-7Md and those of mutant enzymes thereof were evaluated after heat treatment at 55°C for 60 minutes (Table 6).

**[Table 6]**

| Name of mutant | Amino acid substitution introduced into LlLuc-7Md | Residual activity (55°C, 60 min) |
|---|---|---|
| LlLuc-7M | | 0.31 |
| LlLuc-7Md | | 0.35 |
| LlLuc-7Md-1 | F17D | 0.77 |
| LlLuc-7Md-2 | F17E | 0.49 |
| LlLuc-7Md-3 | F17K | 0.71 |
| LlLuc-7Md-4 | Y18L | 0.49 |
| LlLuc-7Md-5 | A41V | 0.81 |
| LlLuc-7Md-6 | Y53L | 0.44 |
| LlLuc-7Md-7 | V75K | 0.65 |
| LlLuc-7Md-8 | I110D | 0.64 |
| LlLuc-7Md-9 | I110E | 0.52 |
| LlLuc-7Md-10 | I110K | 0.48 |
| LlLuc-7Md-11 | I110R | 0.56 |
| LlLuc-7Md-12 | V158D | 0.37 |
| LlLuc-7Md-13 | V158E | 0.39 |
| LlLuc-7Md-14 | H223V | 0.48 |
| LlLuc-7Md-15 | H2231 | 0.78 |
| LlLuc-7Md-16 | H223L | 0.61 |
| LlLuc-7Md-17 | A224I | 0.40 |
| LlLuc-7Md-18 | Y229F | 0.38 |
| LlLuc-7Md-19 | Y229L | 0.57 |
| LlLuc-7Md-20 | Y257F | 0.48 |
| LlLuc-7Md-21 | Y306F | 0.42 |
| LlLuc-7Md-22 | V312R | 0.49 |
| LlLuc-7Md-23 | V312E | 0.42 |
| LlLuc-7Md-24 | V312K | 0.47 |
| LlLuc-7Md-25 | Y427F | 0.64 |

The residual activities of the LlLuc-7M mutants shown in Table 6 were increased by 0.04 to 0.50, compared with that of LlLuc-7M. In other words, the residual activities of the LlLuc-7M mutants were enhanced by 13% to 161% and this indicates that all the LlLuc-7M mutants exhibited improved thermostability. It is highly plausible for such mutations to improve the thermostability in the same manner when such mutations are introduced into luciferases from other sources.

The residual activity of LlLuc-7Md and those of mutant enzymes thereof after heat treatment at 60°C for 30 minutes are shown in the table (Table 7).

**[Table 7]**

| Name of mutant | Amino acid substitution introduced into LlLuc-7Md | Residual activity (60°C, 30 min) |
|---|---|---|
| LlLuc-7Md | | 0.01 |
| LlLuc-8Md | V75K | 0.03 |
| LlLuc-9Md | V75K, I110D | 0.30 |
| LlLuc-10Md | V75K, I110D, V312K | 0.42 |
| LlLuc-11Md | F17E, V75K, I110D, V312K | 0.49 |
| LlLuc-12Md | F17E, V75K, I110D, V158D, V312K | 0.52 |
| LlLuc-13Md | F17E, A41V, V75K, I110D, V158D, V312K | 0.64 |
| LlLuc-14Md | F17E, A41V, V75K, I110D, V158D, Y229L, V312K | 0.70 |
| LlLuc-15Md | F17E, A41V, V75K, I110D, V158D, Y229L, Y257F, V312K | 0.82 |
| LlLuc-16Md | F17E, A41V, Y53L, V75K, I110D, V158D, Y229L, Y257F, V312K | 0.92 |
| LlLuc-15Md-2 | F17E, A41V, Y53L, V75K, I110D, V158D, Y257F, V312K | 1.02 |

The residual activities of LlLuc-8Md to LlLuc-16Md and LlLuc-15Md-2 mutants shown in Table 7 were increased by 0.02 to 1.01, compared with that of LlLuc-7Md. In other words, the residual activities were enhanced by 200% to 10100% and this indicates that thermostability of the LlLuc-7Md mutants were enhanced drastically by combining the amino acid substitutions shown in Table 6.

### Industrial Applicability

The present disclosure provides a luciferase having improved thermostability. The present disclosure also provides a luciferase having significantly improved thermostability. The present disclosure further provides a luciferase having improved affinity to a substrate. Such luciferases can be used for ATP measurement, ATP detection, luminescence measurement, and other purposes.

Various literatures including patent applications and manufacturers' manuals are cited in the present specification. While the disclosure of these literatures are not to be interpreted as being related to the patentability of the present disclosure, the disclosure of these literatures is incorporated herein by reference in their entirety. More specifically, all references are incorporated herein by reference, as in the case of specifically and individually stating that each literature is incorporated by reference.

### Brief Description of Sequences

SEQ ID NO: 1: luciferase from Luciola lateralis
SEQ ID NO: 2: mutated luciferase from L. lateralis (the same may be referred to as "HLK-C393L," see WO 2020/009215)
SEQ ID NO: 3: nucleotide sequence of the gene encoding SEQ ID NO: 2
SEQ ID NOs: 4 to 9: sequences of primers for introducing mutations
SEQ ID NO: 10: mutated luciferase from L. lateralis (LlLuc-4M)
SEQ ID NO: 11: nucleotide sequence of the gene encoding SEQ ID NO: 10
SEQ ID NOs: 12 to 26: sequences of primers for introducing mutations
SEQ ID NO: 27: mutated luciferase from L. lateralis (LlLuc-7Md)
SEQ ID NO: 28: nucleotide sequence of the gene encoding SEQ ID NO: 27
SEQ ID NOs: 29 to 68: sequences of primers for introducing mutations
SEQ ID NO: 69: amino acid sequence of Luciola cruciata luciferase
SEQ ID NO: 70: nucleotide sequence of Luciola cruciata luciferase
SEQ ID NO: 71: amino acid sequence of Photinus pyralis luciferase
SEQ ID NO: 72: nucleotide sequence of Photinus pyralis luciferase
SEQ ID NO: 73: amino acid sequence of Photuris pennsylvanica luciferase
SEQ ID NO: 74: nucleotide sequence of Photuris pennsylvanica luciferase

### Sequences

SEQ ID NO: 1: amino acid sequence of luciferase from Luciola lateralis 
MENMENDENIVYGPEPFYPIEEGSAGAQLRKYMDRYAKLGAIAFTNALTG
VDYTYAEYLEKSCCLGEALKNYGLVVDGRIALCSENCEEFFIPVLAGLFI
GVGVAPTNEIYTLRELVHSLGISKPTIVFSSKKGLDKVITVQKTVTAIKT
IVILDSKVDYRGYQSMDNFIKKNTPQGFKGSSFKTVEVNRKEQVALIMNS
SGSTGLPKGVQLTHENAVTRFSHARDPIYGNQVSPGTAILTVVPFHHGFG
 
MFTTLGYLTCGFRIVMLTKFDEETFLKTLQDYKCSSVILVPTLFAILNRS
ELLDKYDLSNLVEIASGGAPLSKEIGEAVARRFNLPGVRQGYGLTETTSA
IIITPEGDDKPGASGKVVPLFKAKVIDLDTKKTLGPNRRGEVCVKGPMLM
KGYVDNPEATREIIDEEGWLHTGDIGYYDEEKHFFIVDRLKSLIKYKGYQ
VPPAELESVLLQHPNIFDAGVAGVPDPIAGELPGAVVVLEKGKSMTEKEV
MDYVASQVSNAKRLRGGVRFVDEVPKGLTGKIDGKAIREILKKPVAKM
SEQ ID NO: 2: amino acid sequence of mutated luciferase from Luciola lateralis (the same may be referred to as "HLK-C393L") 
MENMENDENIVYGPEPFYPIEEGSAGAQLRKYMDRYAKLGAIAFTNALTG
VDYTYAEYLEKSCCLGEALKNYGLVVDGRIALCSENCEEFFIPVLAGLFI
GVGVAPTNEIYTLRELVHSLGISKPTIVFSSKKGLDKVITVQKTVTAIKT
IVILDSKVDYRGYQSMDNFIKKNTPQGFKGSSFKTVEVNRKEQVALIMNS
SGSTGLPKGVQLTHENLVTRFSHARDPIYGNQVSPGTAILTVVPFHHGFG
MFTTLGYLTCGFRIVMLTKFDEETFLKTLQDYKCSSVILVPTLFAILNRS
ELLDKYDLSNLVEIASGGAPLSKEIGEAVARRFNLPGVRQGYGLTETTSA
IIITPEGDDKPGASGKVVPLFKAKVIDLDTKKTLGPNRRGEVLVKGPMLM
KGYVDNPEATREIIDEEGWLHTGDIGYYDEEKHFFIVDRLKSLIKYKGYQ
VPPAELESVLLQHPNIFDAGVAGVPDPIAGELPGAVVVLKKGKSMTEKEV
MDYVASQVSNAKRLRGGVRFVDEVPKGLTGKIDGKAIREILKKPVAKM
SEQ ID NO: 3: nucleotide sequence of the gene of mutant luciferase from Luciola lateralis (it may be referred to as "HLK-C393L") 
atggaaaacatggagaacgatgaaaatattgtgtatggtcctgaaccattttaccctatt
gaagagggatctgctggagcacaattgcgcaagtatatggatcgatatgcaaaacttgga
gcaattgcttttactaacgcacttaccggtgtcgattatacgtacgccgaatacttagaa
aaatcatgctgtctaggagaggctttaaagaattatggtttggttgttgatggaagaatt
gcgttatgcagtgaaaactgtgaagagttctttattcctgtattagccggtttatttata
ggtgtcggtgtggctccaactaatgagatttacactctacgtgaattggttcacagttta
ggcatctctaagccaacaattgtatttagttctaaaaaaggattagataaagttataact
 
gtacaaaaaacggtaactgctattaaaaccattgttatattggacagcaaagtggattat
agaggttatcaatccatggacaactttattaaaaaaaacactccacaaggtttcaaagga
tcaagttttaaaactgtagaagttaaccgcaaagaacaagttgctcttataatgaactct
tcgggttcaaccggtttgccaaaaggtgtgcaacttactcatgaaaatttggtcacgcgt
ttttctcacgctagagatccaatttatggaaaccaagtttcaccaggcacggctatttta
actgtagtaccattccatcatggttttggtatgtttactactttaggctatctaacttgt
ggttttcgtattgtcatgttaacgaaatttgacgaagagacttttttaaaaacactgcaa
gattacaaatgttcaagcgttattcttgtaccgactttgtttgcaattcttaatagaagt
gaattactcgataaatatgatttatcaaatttagttgaaattgcatctggcggagcacct
ttatctaaagaaattggtgaagctgttgctagacgttttaatttaccgggtgttcgtcaa
ggctatggtttaacagaaacaacctctgcaattattatcacaccggaaggcgatgataaa
ccaggtgcttctggcaaagttgtgccattatttaaagcaaaagttatcgatcttgatact
aaaaaaactttgggcccgaacagacgtggagaagttctggtaaagggtcctatgcttatg
aaaggttatgtagataatccagaagcaacaagagaaatcatagatgaagaaggttggttg
cacacaggagatattgggtattacgatgaagaaaaacatttctttatcgtggatcgtttg
aagtctttaatcaaatacaaaggatatcaagtaccacctgctgaattagaatctgttctt
ttgcaacatccaaatatttttgatgccggcgttgctggcgttccagatcctatagctggt
gagcttccgggagctgttgttgtacttaagaaaggaaaatctatgactgaaaaagaagta
atggattacgttgctagtcaagtttcaaatgcaaaacgtttgcgtggtggtgtccgtttt
gtggacgaagtacctaaaggtctcactggtaaaattgacggtaaagcaattagagaaata
ctgaagaaaccagttgctaagatgtaa
SEQ ID NO: 4: the primer for introducing G326S
   aatttctttagataaaggtgctccgccaga
SEQ ID NO: 5: the primer for introducing G326S
   tctaaagaaattagcgaagctgttgctaga
SEQ ID NO: 6: the primer for introducing V392I
   ttctccacgtctgttcgggcccaaagtttt
SEQ ID NO: 7: the primer for introducing V392I
   agacgtggagaaattctggtaaagggtcct
SEQ ID NO: 8: the primer for introducing F467I
   aatatttggatgttgcaaaagaacagattc
SEQ ID NO: 9: the primer for introducing F467I
   catccaaatattattgatgccggcgttgct
SEQ ID NO: 10: amino acid sequence of mutant luciferase from Luciola lateralis (LlLuc-4M) 
MENMENDENIVYGPEPFYPIEEGSAGAQLRKYMDRYAKLGAIAFTNALTG
VDYTYAEYLEKSCCLGEALKNYGLVVDGRIALCSENCEEFFIPVLAGLFI
GVGVAPTNEIYTLRELVHSLGISKPTIVFSSKKGLDKVITVQKTVTAIKT
IVILDSKVDYRGYQSMDNFIKKNTPQGFKGSSFKTVEVNRKEQVALIMNS
SGSTGLPKGVQLTHENLVTRFSHARDPIYGNQVSPGTAILTVVPFHHGFG
MFTTLGYLTCGFRIVMLTKFDEETFLKTLQDYKCSSVILVPTLFAILNRS
ELLDKYDLSNLVEIASGGAPLSKEISEAVARRFNLPGVRQGYGLTETTSA
IIITPEGDDKPGASGKVVPLFKAKVIDLDTKKTLGPNRRGEILVKGPMLM
KGYVDNPEATREIIDEEGWLHTGDIGYYDEEKHFFIVDRLKSLIKYKGYQ
VPPAELESVLLQHPNIIDAGVAGVPDPIAGELPGAVVVLKKGKSMTEKEV
MDYVASQVSNAKRLRGGVRFVDEVPKGLTGKIDGKAIREILKKPVAKM
SEQ ID NO: 11: nucleotide sequence of the gene of mutant luciferase from Luciola lateralis (L1Luc-4M) 
atggaaaacatggagaacgatgaaaatattgtgtatggtcctgaaccattttaccctatt

gaagagggatctgctggagcacaattgcgcaagtatatggatcgatatgcaaaacttgga

gcaattgcttttactaacgcacttaccggtgtcgattatacgtacgccgaatacttagaa

aaatcatgctgtctaggagaggctttaaagaattatggtttggttgttgatggaagaatt

gcgttatgcagtgaaaactgtgaagagttctttattcctgtattagccggtttatttata

ggtgtcggtgtggctccaactaatgagatttacactctacgtgaattggttcacagttta

ggcatctctaagccaacaattgtatttagttctaaaaaaggattagataaagttataact

gtacaaaaaacggtaactgctattaaaaccattgttatattggacagcaaagtggattat
 
agaggttatcaatccatggacaactttattaaaaaaaacactccacaaggtttcaaagga
tcaagttttaaaactgtagaagttaaccgcaaagaacaagttgctcttataatgaactct
tcgggttcaaccggtttgccaaaaggtgtgcaacttactcatgaaaatttggtcacgcgt
ttttctcacgctagagatccaatttatggaaaccaagtttcaccaggcacggctatttta
actgtagtaccattccatcatggttttggtatgtttactactttaggctatctaacttgt
ggttttcgtattgtcatgttaacgaaatttgacgaagagacttttttaaaaacactgcaa
gattacaaatgttcaagcgttattcttgtaccgactttgtttgcaattcttaatagaagt
gaattactcgataaatatgatttatcaaatttagttgaaattgcatctggcggagcacct
ttatctaaagaaattagcgaagctgttgctagacgttttaatttaccgggtgttcgtcaa
ggctatggtttaacagaaacaacctctgcaattattatcacaccggaaggcgatgataaa
ccaggtgcttctggcaaagttgtgccattatttaaagcaaaagttatcgatcttgatact
aaaaaaactttgggcccgaacagacgtggagaaattctggtaaagggtcctatgcttatg
aaaggttatgtagataatccagaagcaacaagagaaatcatagatgaagaaggttggttg
cacacaggagatattgggtattacgatgaagaaaaacatttctttatcgtggatcgtttg
aagtctttaatcaaatacaaaggatatcaagtaccacctgctgaattagaatctgttctt
ttgcaacatccaaatattattgatgccggcgttgctggcgttccagatcctatagctggt
gagcttccgggagctgttgttgtacttaagaaaggaaaatctatgactgaaaaagaagta
atggattacgttgctagtcaagtttcaaatgcaaaacgtttgcgtggtggtgtccgtttt
gtggacgaagtacctaaaggtctcactggtaaaattgacggtaaagcaattagagaaata
ctgaagaaaccagttgctaagatgtaa
SEQ ID NO: 12: the primer for introducing F90Y
   ctcttcacagttttcactgcataacgcaat
SEQ ID NO: 13: the primer for introducing F90Y
   aactgtgaagagtactttattcctgtatta
SEQ ID NO: 14: the primer for introducing F221L
   acgcgtgaccaaattctcgtgagtaagttg
SEQ ID NO: 15: the primer for introducing F221L
   ttggtcacgcgtttatctcacgccagagat
SEQ ID NO: 16: the primer for introducing F252L
   cataccaaaaccatggtgaaatgggactac
SEQ ID NO: 17: the primer for introducing F252L
   ggttttggtatgttaactactttaggctat
SEQ ID NO: 18: the primer for introducing F262Y
   accacaagttagatagcctaaagtagtaaa
SEQ ID NO: 19: the primer for introducing F262Y
   ctaacttgtggttatcgtattgtcatgtta
SEQ ID NO: 20: the primer for introducing F371L
   taatggcacaaccttaccagaagcacctgg
SEQ ID NO: 21: the primer for introducing F371L
   gttgtgccattattgaaagcaaaagttatc
SEQ ID NO: 22: the primer for introducing F294L
   caaagtcggtacaagaataacgcttgaaca
SEQ ID NO: 23: the primer for introducing F294L
   gtaccgactttgctggcaattcttaataga
SEQ ID NO: 24: the primer for introducing F252/F262Y
   accacaagttagatagcctaaagtagttaa
SEQ ID NO: 25: the primer for deleting P544-M548
   tttcttcagtatttctctaattgctttacc
SEQ ID NO: 26: the primer for deleting P544-M548
   taaggatccggctgctaacaaagcccgaaa
SEQ ID NO: 27: mutant luciferase from Luciola lateralis (LlLuc-7Md) 
MENMENDENIVYGPEPFYPIEEGSAGAQLRKYMDRYAKLGAIAFTNALTG
VDYTYAEYLEKSCCLGEALKNYGLVVDGRIALCSENCEEFFIPVLAGLFI
GVGVAPTNEIYTLRELVHSLGISKPTIVFSSKKGLDKVITVQKTVTAIKT
IVILDSKVDYRGYQSMDNFIKKNTPQGFKGSSFKTVEVNRKEQVALIMNS
SGSTGLPKGVQLTHENLVTRFSHARDPIYGNQVSPGTAILTVVPFHHGFG
MLTTLGYLTCGYRIVMLTKFDEETFLKTLQDYKCSSVILVPTLLAILNRS
ELLDKYDLSNLVEIASGGAPLSKEIGEAVARRFNLPGVRQGYGLTETTSA
IIITPEGDDKPGASGKVVPLFKAKVIDLDTKKTLGPNRRGEVLVKGPMLM
KGYVDNPEATREIIDEEGWLHTGDIGYYDEEKHFFIVDRLKSLIKYKGYQ
VPPAELESVLLQHPNIFDAGVAGVPDPIAGELPGAVVVLKKGKSMTEKEV
MDYVASQVSNAKRLRGGVRFVDEVPKGLTGKIDGKAIREILKK
SEQ ID NO: 28: nucleotide sequence of the gene of mutant luciferase from Luciola lateralis (LlLuc-7Md) 
atggaaaacatggagaacgatgaaaatattgtgtatggtcctgaaccattttaccctatt

gaagagggatctgctggagcacaattgcgcaagtatatggatcgatatgcaaaacttgga

gcaattgcttttactaacgcacttaccggtgtcgattatacgtacgccgaatacttagaa

aaatcatgctgtctaggagaggctttaaagaattatggtttggttgttgatggaagaatt

gcgttatgcagtgaaaactgtgaagagttctttattcctgtattagccggtttatttata

ggtgtcggtgtggctccaactaatgagatttacactctacgtgaattggttcacagttta

ggcatctctaagccaacaattgtatttagttctaaaaaaggattagataaagttataact

gtacaaaaaacggtaactgctattaaaaccattgttatattggacagcaaagtggattat

agaggttatcaatccatggacaactttattaaaaaaaacactccacaaggtttcaaagga

tcaagttttaaaactgtagaagttaaccgcaaagaacaagttgctcttataatgaactct

tcgggttcaaccggtttgccaaaaggtgtgcaacttactcatgaaaatttggtcacgcgt

ttttctcacgctagagatccaatttatggaaaccaagtttcaccaggcacggctatttta

actgtagtcccatttcaccatggttttggtatgttaactactttaggctatctaacttgt

ggttatcgtattgtcatgttaacgaaatttgacgaagagacttttttaaaaacactgcaa

gattacaaatgttcaagcgttattcttgtaccgactttgctggcaattcttaatagaagt

gaattactcgataaatatgatttatcaaatttagttgaaattgcatctggcggagcacct
 
ttatctaaagaaattagcgaagctgttgctagacgttttaatttaccgggtgttcgtcaa
ggctatggtttaacagaaacaacctctgcaattattatcacaccggaaggcgatgataaa
ccaggtgcttctggcaaagttgtgccattatttaaagcaaaagttatcgatcttgatact
aaaaaaactttgggcccgaacagacgtggagaaattctggtaaagggtcctatgcttatg
aaaggttatgtagataatccagaagcaacaagagaaatcatagatgaagaaggttggttg
cacacaggagatattgggtattacgatgaagaaaaacatttctttatcgtggatcgtttg
aagtctttaatcaaatacaaaggatatcaagtaccacctgctgaattagaatctgttctt
ttgcaacatccaaatattattgatgccggcgttgctggcgttccagatcctatagctggt
gagcttccgggagctgttgttgtacttaagaaaggaaaatctatgactgaaaaagaagta
atggattacgttgctagtcaagtttcaaatgcaaaacgtttgcgtggtggtgtccgtttt
gtggacgaagtacctaaaggtctcactggtaaaattgacggtaaagcaattagagaaata
ctgaagaaataa
SEQ ID NO: 29: primer for introducing F17D/E/K
   tggttcaggaccatacacaatattttcatc
SEQ ID NO: 30: primer for introducing F17D
   ggtcctgaaccagattaccctattgaagag
SEQ ID NO: 31: primer for introducing F17E
   ggtcctgaaccagaataccctattgaagag
SEQ ID NO: 32: primer for introducing F17K
   ggtcctgaaccaaagtaccctattgaagag
SEQ ID NO: 33: primer for introducing Y18L
   aaatggttcaggaccatacacaatattttc
SEQ ID NO: 34: primer for introducing Y18L
   cctgaaccatttttacctattgaagaggga
SEQ ID NO: 35: primer for introducing A41V
   tccaagttttgcatatcgatccatatactt
SEQ ID NO: 36: primer for introducing A41V
   gcaaaacttggagtaattgcttttactaac
SEQ ID NO: 37: primer for introducing Y53L
   atc gacaccggtaagtgcgttagtaaaagc
SEQ ID NO: 38: primer for introducing Y53L
   accggtgtcgatttaacgtacgccgaatac
SEQ ID NO: 39: primer for introducing I110D/E/K/R
   ctcattagttggagccacaccgacacctat
SEQ ID NO: 40: primer for introducing I110D
   ccaactaatgaggattacactctacgtgaa
SEQ ID NO: 41: primer for introducing I110E
   ccaactaatgaggaatacactctacgtgaa
SEQ ID NO: 42: primer for introducing I110K
   ccaactaatgagaagtacactctacgtgaa
SEQ ID NO: 43: primer for introducing I110R
   ccaactaatgagcgttacactctacgtgaa
SEQ ID NO: 44: primer for introducing V158D/E
   tttgctgtccaatataacaatggttttaat
SEQ ID NO: 45: primer for introducing V158D
   ttggacagcaaagatgattatagaggttat
SEQ ID NO: 46: primer for introducing V158E
   ttggacagcaaagaggattatagaggttat
SEQ ID NO: 47: primer for introducing H223V/I/L
   agaaaaacgcgtgaccaaattttcatgagt
SEQ ID NO: 48: primer for introducing H223V
   acgcgtttttctgtcgctagagatccaatt
SEQ ID NO: 49: primer for introducing H223I
   acgcgtttttctatcgctagagatccaatt
SEQ ID NO: 50: primer for introducing H223L
   acgcgtttttctctcgctagagatccaatt
SEQ ID NO: 51: primer for introducing A224I
   gtgagaaaaacgcgtgaccaaattttcatg
SEQ ID NO: 52: primer for introducing A224I
   cgtttttctcacattagagatccaatttat
SEQ ID NO: 53: primer for introducing Y229F/L
   aattggatctctagcgtgagaaaaacgcgt
SEQ ID NO: 54: primer for introducing Y229F
   agagatccaatttttggaaaccaagtttca
SEQ ID NO: 55: primer for introducing Y229L
   agagatccaattttaggaaaccaagtttca
SEQ ID NO: 56: primer for introducing Y257F
   gcctaaagtagttaacataccaaaaccatg
SEQ ID NO: 57: primer for introducing Y257F
   actactttaggctttctaacttgtggttat
SEQ ID NO: 58: primer for introducing Y306F
   tttatcgagtaattcacttctattaagaat
SEQ ID NO: 59: primer for introducing Y306F
   ttactcgataaattcgatttatcaaattta
SEQ ID NO: 60: primer for introducing V312D/E/K
   taaatttgataaatcatatttatcgagtaa
SEQ ID NO: 61: primer for introducing V312R
   ttatcaaatttacgtgaaattgcatctggc
SEQ ID NO: 62: primer for introducing V312E
   ttatcaaatttagaagaaattgcatctgge
SEQ ID NO: 63: primer for introducing V312K
   ttatcaaatttaaaggaaattgcatctgge
SEQ ID NO: 64: primer for introducing Y427F
   cccaatatctcctgtgtgcaaccaaccttc
SEQ ID NO: 65: primer for introducing Y427F
   ggagatattgggttttacgatgaagaaaaa
SEQ ID NO: 66: primer for introducing V75K
   caaaccataattctttaaagcctctcctag
SEQ ID NO: 67: primer for introducing V75K
   aattatggtttgaaagttgatggaagaatt
SEQ ID NO: 68: primer for introducing L229Y (Y229L reversion)
   agagatccaatttatggaaaccaagtttca
SEQ ID NO: 69: amino acid sequence of Luciola cruciata luciferase Met Glu Asn Met Glu Asn Asp Glu Asn Ile Val Gly Pro Lys Pro Phe Tyr Pro Ile Glu Glu Gly Ser Ala Gly Thr Gln Leu Arg Lys Tyr Met Glu Arg Tyr Ala Lys Leu Gly Ala Ile Ala Phe Thr Asn Ala Val Thr Gly Val Asp Tyr Ser Tyr Ala Glu Tyr Leu Glu Lys Ser Cys Cys Leu Gly Lys Ala Leu Gln Asn Tyr Gly Leu Val Val Asp Gly Arg Ile Ala Leu Cys Ser Glu Asn Cys Glu Glu Phe Phe Ile Pro Val Ile Ala Gly Leu Phe Ile Gly Val Gly Val Ala Pro Thr Asn Glu Ile Tyr Thr Leu Arg Glu Leu Val His Ser Leu Gly Ile Ser Lys Pro Thr Ile Val Phe Ser Ser Lys Lys Gly Leu Asp Lys Val Ile Thr Val Gln Lys Thr Val Thr Thr Ile Lys Thr Ile Val Ile Leu Asp Ser Lys Val Asp Tyr Arg Gly Tyr Gln Cys Leu Asp Thr Phe Ile Lys Arg Asn Thr Pro Pro Gly Phe Gln Ala Ser Ser Phe Lys Thr Val Glu Val Asp Arg Lys Glu Gln Val Ala Leu Ile Met Asn Ser Ser Gly Ser Thr Gly Leu Pro Lys Gly Val Gln Leu Thr His Glu Asn Thr Val Thr Arg Phe Ser His Ala Arg Asp Pro Ile Tyr Gly Asn Gln Val Ser Pro Gly Thr Ala Val Leu Thr Val Val Pro Phe His His Gly Phe Gly Met Phe Thr Thr Leu Gly Tyr Leu Ile Cys Gly Phe Arg Val Val Met Leu Thr Lys Phe Asp Glu Glu Thr Phe Leu Lys Thr Leu Gln Asp Tyr Lys Cys Thr Ser Val Ile Leu Val Pro Thr Leu Phe Ala Ile Leu Asn Lys Ser Glu Leu Leu Asn Lys Tyr Asp Leu Ser Asn Leu Val Glu Ile Ala Ser Gly Gly Ala Pro Leu Ser Lys Glu Val Gly Glu Ala Val Ala Arg Arg Phe Asn Leu Pro Gly Val Arg Gln Gly Tyr Gly Leu Thr Glu Thr Thr Ser Ala Ile Ile Ile Thr Pro Glu Gly Asp Asp Lys Pro Gly Ala Ser Gly Lys Val Val Pro Leu Phe Lys Ala Lys Val Ile Asp Leu Asp Thr Lys Lys Ser Leu Gly Pro Asn Arg Arg Gly Glu Val Cys Val Lys Gly Pro Met Leu Met Lys Gly Tyr Val Asn Asn Pro Glu Ala Thr Lys Glu Leu Ile Asp Glu Glu Gly Trp Leu His Thr Gly Asp Ile Gly Tyr Tyr Asp Glu Glu Lys His Phe Phe Ile Val Asp Arg Leu Lys Ser Leu Ile Lys Tyr Lys Gly Tyr Gln Val Pro Pro Ala Glu Leu Glu Ser Val Leu Leu Gln His Pro Ser Ile Phe Asp Ala Gly Val Ala Gly Val Pro Asp Pro Val Ala Gly Glu Leu Pro Gly Ala Val Val Val Leu Glu Ser Gly Lys Asn Met Thr Glu Lys Glu Val Met Asp Tyr Val Ala Ser Gln Val Ser Asn Ala Lys Arg Leu Arg Gly Gly Val Arg Phe Val Asp Glu Val Pro Lys Gly Leu Thr Gly Lys Ile Asp Gly Arg Ala Ile Arg Glu Ile Leu Lys Lys Pro Val Ala Lys Met
SEQ ID NO: 70: nucleotide sequence of Luciola cruciata luciferase 
atggaaaaca tggaaaacga tgaaaatatt gtagttggac ctaaaccgtt ttaccctatc
gaagagggat ctgctggaac acaattacgc aaatacatgg agcgatatgc aaaacttggc
gcaattgctt ttacaaatgc agttactggt gttgattatt cttacgccga atacttggag
aaatcatgtt gtctaggaaa agctttgcaa aattatggtt tggttgttga tggcagaatt
 
gcgttatgca gtgaaaactg tgaagaattt tttattcctg taatagccgg actgtttata
ggtgtaggtg ttgcacccac taatgagatt tacactttac gtgaactggt tcacagttta
ggtatctcta aaccaacaat tgtatttagt tctaaaaaag gcttagataa agttataaca
gtacagaaaa cagtaactac tattaaaacc attgttatac tagatagcaa agttgattat
cgaggatatc aatgtctgga cacctttata aaaagaaaca ctccaccagg ttttcaagca
tccagtttca aaactgtgga agttgaccgt aaagaacaag ttgctcttat aatgaactct
tcgggttcta ccggtttgcc aaaaggcgta caacttactc acgaaaatac agtcactaga
ttttctcatg ctagagatcc gatttatggt aaccaagttt caccaggcac cgctgtttta
actgtcgttc cattccatca tggttttggt atgttcacta ctctagggta tttaatttgt
ggttttcgtg ttgtaatgtt aacaaaattc gatgaagaaa cattttttaa aactctacaa
gattataaat gtacaagtgt tattcttgta ccgaccttgt ttgcaattct caacaaaagt
gaattactca ataaatacga tttgtcaaat ttagttgaga ttgcatctgg cggagcacct
ttatcaaaag aagttggtga agctgttgct agacgcttta atcttcccgg tgttcgtcaa
ggttatggtt taacagaaac aacatctgcc attattatta caccagaagg agacgataaa
ccaggagctt ctggaaaagt cgtgccgttg tttaaagcaa aagttattga tcttgatacc
aaaaaatctt taggtcctaa cagacgtgga gaagtttgtg ttaaaggacc tatgcttatg
aaaggttatg taaataatcc agaagcaaca aaagaactta ttgacgaaga aggttggctg
cacaccggag atattggata ttatgatgaa gaaaaacatt tctttattgt cgatcgtttg
aagtctttaa tcaaatacaa aggataccaa gtaccacctg ccgaattaga atccgttctt
ttgcaacatc catctatctt tgatgctggt gttgccggcg ttcctgatcc tgtagctggc
gagcttccag gagccgttgt tgtactggaa agcggaaaaa atatgaccga aaaagaagta
atggattatg ttgcaagtca agtttcaaat gcaaaacgtt tacgtggtgg tgttcgtttt
gtggatgaag tacctaaagg tcttactgga aaaattgacg gcagagcaat tagagaaatc
cttaagaaac cagttgctaa gatgtga
SEQ ID NO: 71: amino acid sequence of Photinus pyralis luciferase Met Glu Asp Ala Lys Asn Ile Lys Lys Gly Pro Ala Pro Phe Tyr Pro Leu Glu Asp Gly Thr Ala Gly Glu Gln Leu His Lys Ala Met Lys Arg Tyr Ala Leu Val Pro Gly Thr Ile Ala Phe Thr Asp Ala His Ile Glu Val Asn Ile Thr Tyr Ala Glu Tyr Phe Glu Met Ser Val Arg Leu Ala Glu Ala Met Lys Arg Tyr Gly Leu Asn Thr Asn His Arg Ile Val Val Cys Ser Glu Asn Ser Leu Gln Phe Phe Met Pro Val Leu Gly Ala Leu Phe Ile Gly Val Ala Val Ala Pro Ala Asn Asp Ile Tyr Asn Glu Arg Glu Leu Leu Asn Ser Met Asn Ile Ser Gln Pro Thr Val Val Phe Val Ser Lys Lys Gly Leu Gln Lys Ile Leu Asn Val Gln Lys Lys Leu Pro Ile Ile Gln Lys Ile Ile Ile Met Asp Ser Lys Thr Asp Tyr Gln Gly Phe Gln Ser Met Tyr Thr Phe Val Thr Ser His Leu Pro Pro Gly Phe Asn Glu Tyr Asp Phe Val Pro Glu Ser Phe Asp Arg Asp Lys Thr Ile Ala Leu Ile Met Asn Ser Ser Gly Ser Thr Gly Leu Pro Lys Gly Val Ala Leu Pro His Arg Thr Ala Cys Val Arg Phe Ser His Ala Arg Asp Pro Ile Phe Gly Asn Gln Ile Ile Pro Asp Thr Ala Ile Leu Ser Val Val Pro Phe His His Gly Phe Gly Met Phe Thr Thr Leu Gly Tyr Leu Ile Cys Gly Phe Arg Val Val Leu Met Tyr Arg Phe Glu Glu Glu Leu Phe Leu Arg Ser Leu Gln Asp Tyr Lys Ile Gln Ser Ala Leu Leu Val Pro Thr Leu Phe Ser Phe Phe Ala Lys Ser Thr Leu Ile Asp Lys Tyr Asp Leu Ser Asn Leu His Glu Ile Ala Ser Gly Gly Ala Pro Leu Ser Lys Glu Val Gly Glu Ala Val Ala Lys Arg Phe His Leu Pro Gly Ile Arg Gln Gly Tyr Gly Leu Thr Glu Thr Thr Ser Ala Ile Leu Ile Thr Pro Glu Gly Asp Asp Lys Pro Gly Ala Val Gly Lys Val Val Pro Phe Phe Glu Ala Lys Val Val Asp Leu Asp Thr Gly Lys Thr Leu Gly Val Asn Gln Arg Gly Glu Leu Cys Val Arg Gly Pro Met Ile Met Ser Gly Tyr Val Asn Asn Pro Glu Ala Thr Asn Ala Leu Ile Asp Lys Asp Gly Trp Leu His Ser Gly Asp Ile Ala Tyr Trp Asp Glu Asp Glu His Phe Phe Ile Val Asp Arg Leu Lys Ser Leu Ile Lys Tyr Lys Gly Tyr Gln Val Ala Pro Ala Glu Leu Glu Ser Ile Leu Leu Gln His Pro Asn Ile Phe Asp Ala Gly Val Ala Gly Leu Pro Asp Asp Asp Ala Gly Glu Leu Pro Ala Ala Val Val Val Leu Glu His Gly Lys Thr Met Thr Glu Lys Glu Ile Val Asp Tyr Val Ala Ser Gln Val Thr Thr Ala Lys Lys Leu Arg Gly Gly Val Val Phe Val Asp Glu Val Pro Lys Gly Leu Thr Gly Lys Leu Asp Ala Arg Lys Ile Arg Glu Ile Leu Ile Lys Ala Lys Lys Gly Gly Lys Ser Lys Leu
SEQ ID NO: 72: nucleotide sequence of Photinus pyralis luciferase 
atggaagacg ccaaaaacat aaagaaaggc ccggcgccat tctatccgct agaggatgga
accgctggag agcaactgca taaggctatg aagagatacg ccctggttcc tggaacaatt
gcttttacag atgcacatat cgaggtgaac atcacgtacg cggaatactt cgaaatgtcc
gttcggttgg cagaagctat gaaacgatat gggctgaata caaatcacag aatcgtcgta
tgcagtgaaa actctcttca attctttatg ccggtgttgg gcgcgttatt tatcggagtt
gcagttgcgc ccgcgaacga catttataat gaacgtgaat tgctcaacag tatgaacatt
tcgcagccta ccgtagtgtt tgtttccaaa aaggggttgc aaaaaatttt gaacgtgcaa
aaaaaattac caataatcca gaaaattatt atcatggatt ctaaaacgga ttaccaggga
tttcagtcga tgtacacgtt cgtcacatct catctacctc ccggttttaa tgaatacgat
tttgtaccag agtcctttga tcgtgacaaa acaattgcac tgataatgaa ctcctctgga
tctactgggt tacctaaggg tgtggccctt ccgcatagaa ctgcctgcgt cagattctcg
catgccagag atcctatttt tggcaatcaa atcattccgg atactgcgat tttaagtgtt
gttccattcc atcacggttt tggaatgttt actacactcg gatatttgat atgtggattt
cgagtcgtct taatgtatag atttgaagaa gagctgtttt tacgatccct tcaggattac
aaaattcaaa gtgcgttgct agtaccaacc ctattttcat tcttcgccaa aagcactctg
attgacaaat acgatttatc taatttacac gaaattgctt ctgggggcgc acctctttcg
aaagaagtcg gggaagcggt tgcaaaacgc ttccatcttc cagggatacg acaaggatat
gggctcactg agactacatc agctattctg attacacccg agggggatga taaaccgggc
gcggtcggta aagttgttcc attttttgaa gcgaaggttg tggatctgga taccgggaaa
acgctgggcg ttaatcagag aggcgaatta tgtgtcagag gacctatgat tatgtccggt
tatgtaaaca atccggaagc gaccaacgcc ttgattgaca aggatggatg gctacattct
ggagacatag cttactggga cgaagacgaa cacttcttca tagttgaccg cttgaagtct
ttaattaaat acaaaggata ccaggtggcc cccgctgaat tggagtcgat attgttacaa
caccccaaca tcttcgacgc gggcgtggca ggtcttcccg acgatgacgc cggtgaactt
cccgccgccg ttgttgtttt ggagcacgga aagacgatga cggaaaaaga gatcgtggat
tacgtcgcca gtcaagtaac aaccgccaaa aagttgcgcg gaggagttgt gtttgtggac
gaagtaccga aaggtcttac cggaaaactc gacgcaagaa aaatcagaga gatcctcata
 aaggccaaga agggcggaaa gtccaaattg taa
SEQ ID NO: 73: amino acid sequence of Photuris pennsylvanica luciferase
SEQ ID NO: 74: nucleotide sequence of Photuris pennsylvanica luciferase

atggaagata aaaatatttt atatggacct gaaccatttc atcccttggc tgatgggacg
gctggagaac agatgtttta cgcattatct cggtatgcag atatttcagg atgcattgca
ttgacaaatg ctcatacaaa agaaaatgtt ttatatgaag aatttttaaa attgtcgtgt
cgtttagcgg aaagttttaa aaagtatgga ttaaaacaaa acgacacaat agcggtgtgt
agtgaaaatg gtttgcaatt tttccttcct ttaattgcat cattgtatct tggaataatt
gcagcacctg ttagtgataa atacattgaa cgtgaattaa tacacagtct tggtattgta
aaaccacgca taattttttg ttccaagaat acttttcaaa aagtactgaa tgtaaaatct
aaattaaaat atgtagaaac tattattata ttagacttaa atgaagactt aggaggttat
caatgcctca acaactttat ttctcaaaat tccgatatta atcttgacgt aaagaaattt
aaaccaaatt cttttaatcg agacgatcag gttgcgttgg taatgttttc ttctggtaca
actggtgttt ctaagggagt catgctaact cacaagaata ttgttgcacg attttctcat
tgcaaagatc ctacttttgg taacgcaatt aatccaacga cagcaatttt aacagtaata
ccattccacc atggttttgg tatgactacc acattaggat actttacttg tggattccga
gttgctctaa tgcacacgtt tgaagaaaaa ctatttttac aatcattaca agattataaa
gtggaaagta ctttacttgt accaacatta atggcatttt ttccaaaaag tgcgttagtt
gaaaagtacg atttatcgca cttaaaagaa attgcatctg gtggcgcacc tttatcaaaa
gaaattgggg agatggtgaa aaaacggttt aaattaaact ttgtcaggca agggtatgga
ttaacagaaa ccacttcggc tgttttaatt acaccggaca ctgacgtcag accgggatca
actggtaaaa tagtaccatt tcacgctgtt aaagttgtgg atcctacaac aggaaaaatt
ttggggccaa atgaaactgg agaattgtat tttaaaggcg acatgataat gaaaagttat

tataataatg aagaagctac taaagcaatt attaacaaag acggatggtt gcgctctggt
gatattgctt attatgacaa tgatggccat ttttatattg tggacaggct gaagtcatta
attaaatata aaggttatca ggttgcacct gctgaaattg agggaatact cttacaacac
ccgtatattg ttgatgccgg cgttactggt ataccggatg aagccgcggg cgagcttcca
gctgcaggtg ttgtagtaca gactggaaaa tatctaaacg aacaaatcgt acaaaatttt
gtttccagtc aagtttcaac agccaaatgg ctacgtggtg gggtgaaatt tttggatgaa
attcccaaag gatcaactgg aaaaattgac agaaaagtgt taagacaaat gtttgaaaaa
cacaaatcta agctgtaa

## Claims

1. A luciferase mutant, wherein the luciferase before amino acid substitution has at least 70%, 80%, or 90% amino acid sequence identity to SEQ ID NO: 10, and the luciferase after amino acid substitution has a substitution at the position corresponding to position 252 of SEQ ID NO: 1 with leucine and has improved thermostability, compared to the luciferase before amino acid substitution.

2. The luciferase mutant of Claim 1, wherein the position corresponding to position 262 of SEQ ID NO: 1 is substituted with tyrosine and/or the position corresponding to position 294 of SEQ ID NO: 1 is substituted with leucine.

3. The luciferase mutant of Claim 1 or 2, wherein the amino acid residue at the position corresponding to position 90, 371, 41, 427, 53, 306, or 312 of SEQ ID NO: 1 is substituted.

4. The luciferase mutant of Claim 3, wherein
(a) the amino acid residue at the position corresponding to position 90 of SEQ ID NO: 1 is substituted and the amino acid residue at the position corresponding to position 90 of SEQ ID NO: 1 is an amino acid residue selected from the group consisting of tyrosine, leucine, isoleucine, cysteine, serine, threonine, aspartic acid, glutamic acid, proline, valine, tryptophan, methionine, alanine, glycine, glutamine, asparagine, lysine, histidine, and arginine;
(b) the amino acid residue at the position corresponding to position 371 of SEQ ID NO: 1 is substituted and the amino acid residue at the position corresponding to position 371 of SEQ ID NO: 1 is an amino acid residue selected from the group consisting of leucine, tyrosine, cysteine, threonine, aspartic acid, glutamic acid, isoleucine, proline, valine, tryptophan, methionine, alanine, glycine, glutamine, asparagine, lysine, histidine, and arginine;
(c) the amino acid residue at the position corresponding to position 41 of SEQ ID NO: 1 is substituted and the amino acid residue at the position corresponding to position 41 of SEQ ID NO: 1 is an amino acid residue selected from the group consisting of valine, leucine, isoleucine, methionine, tyrosine, cysteine, serine, threonine, aspartic acid, glutamic acid, tryptophan, phenylalanine, proline, glycine, glutamine, asparagine, lysine, histidine, and arginine;
(d) the amino acid residue at the position corresponding to position 427 of SEQ ID NO: 1 is substituted and the amino acid residue at the position corresponding to position 427 of SEQ ID NO: 1 is an amino acid residue selected from the group consisting of phenylalanine, leucine, isoleucine, cysteine, serine, threonine, aspartic acid, glutamic acid, tryptophan, methionine, valine, alanine, proline, glycine, glutamine, asparagine, lysine, histidine, and arginine;
(e) the amino acid residue at the position corresponding to position 53 of SEQ ID NO: 1 is substituted and the amino acid residue at the position corresponding to position 53 of SEQ ID NO: 1 is an amino acid residue selected from the group consisting of leucine, isoleucine, cysteine, serine, threonine, aspartic acid, glutamic acid, tryptophan, methionine, valine, phenylalanine, alanine, proline, glycine, glutamine, asparagine, lysine, histidine, and arginine;
(f) the amino acid residue at the position corresponding to position 306 of SEQ ID NO: 1 is substituted and the amino acid residue at the position corresponding to position 306 of SEQ ID NO: 1 is an amino acid residue selected from the group consisting of phenylalanine, leucine, isoleucine, cysteine, serine, threonine, aspartic acid, glutamic acid, tryptophan, methionine, valine, alanine, proline, glycine, glutamine, asparagine, lysine, histidine, and arginine; or
(g) the amino acid residue at the position corresponding to position 312 of SEQ ID NO: 1 is substituted and the amino acid residue at the position corresponding to position 312 of SEQ ID NO: 1 is an amino acid residue selected from the group consisting of tyrosine, phenylalanine, leucine, isoleucine, cysteine, serine, threonine, aspartic acid, glutamic acid, tryptophan, methionine, alanine, proline, glycine, glutamine, asparagine, lysine, histidine, and arginine.

5. The luciferase mutant of Claim 4, wherein
(a) the amino acid residue at the position corresponding to position 90 of SEQ ID NO: 1 is substituted and the amino acid residue at the position corresponding to position 90 of SEQ ID NO: 1 is tyrosine;
(b) the amino acid residue at the position corresponding to position 371 of SEQ ID NO: 1 is substituted and the amino acid residue at the position corresponding to position 371 of SEQ ID NO: 1 is leucine;
(c) the amino acid residue at the position corresponding to position 41 of SEQ ID NO: 1 is substituted and the amino acid residue at the position corresponding to position 41 of SEQ ID NO: 1 is valine;
(d) the amino acid residue at the position corresponding to position 427 of SEQ ID NO: 1 is substituted and the amino acid residue at the position corresponding to position 427 of SEQ ID NO: 1 is phenylalanine;
(e) the amino acid residue at the position corresponding to position 53 of SEQ ID NO: 1 is substituted and the amino acid residue at the position corresponding to position 53 of SEQ ID NO: 1 is leucine;
(f) the amino acid residue at the position corresponding to position 306 of SEQ ID NO: 1 is substituted and the amino acid residue at the position corresponding to position 306 of SEQ ID NO: 1 is phenylalanine; or
(g) the amino acid residue at the position corresponding to position 312 of SEQ ID NO: 1 is substituted and the amino acid residue at the position corresponding to position 312 of SEQ ID NO: 1 is glutamic acid, lysine, or arginine.

6. The luciferase mutant of any one of Claims 1 to 5, which further comprises an amino acid sequence in which the amino acid residue at the position corresponding to position 221, 17, 18, 75, 110, 223, 224, 257, 229, or 158 of SEQ ID NO: 1 is substituted.

7. The luciferase mutant of Claim 6, wherein
(i) the amino acid residue at the position corresponding to position 221 of SEQ ID NO: 1 is substituted and the amino acid residue at the position corresponding to position 221 of SEQ ID NO: 1 is an amino acid residue selected from the group consisting of leucine, tyrosine, cysteine, serine, threonine, aspartic acid, glutamic acid, isoleucine, proline, valine, tryptophan, methionine, alanine, glycine, glutamine, asparagine, lysine, histidine, and arginine;
(ii) the amino acid residue at the position corresponding to position 17 of SEQ ID NO: 1 is substituted and the amino acid residue at the position corresponding to position 17 of SEQ ID NO: 1 is an amino acid residue selected from the group consisting of aspartic acid, glutamic acid, lysine, histidine, arginine, tyrosine, cysteine, serine, threonine, tryptophan, methionine, leucine, isoleucine, valine, alanine, proline, glycine, glutamine, and asparagine;
(iii) the amino acid residue at the position corresponding to position 18 of SEQ ID NO: 1 is substituted and the amino acid residue at the position corresponding to position 18 of SEQ ID NO: 1 is an amino acid residue selected from the group consisting of leucine, isoleucine, cysteine, serine, threonine, aspartic acid, glutamic acid, tryptophan, methionine, valine, phenylalanine, alanine, proline, glycine, glutamine, asparagine, lysine, histidine, and arginine;
(iv) the amino acid residue at the position corresponding to position 75 of SEQ ID NO: 1 is substituted and the amino acid residue at the position corresponding to position 75 of SEQ ID NO: 1 is an amino acid residue selected from the group consisting of lysine, histidine, arginine, aspartic acid, glutamic acid, tyrosine, cysteine, serine, threonine, tryptophan, methionine, leucine, isoleucine, phenylalanine, alanine, proline, glycine, glutamine, and asparagine;
(v) the amino acid residue at the position corresponding to position 110 of SEQ ID NO: 1 is substituted and the amino acid residue at the position corresponding to position 110 of SEQ ID NO: 1 is an amino acid residue selected from the group consisting of aspartic acid, glutamic acid, lysine, arginine, histidine, tyrosine, cysteine, serine, threonine, tryptophan, methionine, leucine, valine, phenylalanine, alanine, proline, glycine, glutamine, and asparagine;
(vi) the amino acid residue at the position corresponding to position 223 of SEQ ID NO: 1 is substituted and the amino acid residue at the position corresponding to position 223 of SEQ ID NO: 1 is an amino acid residue selected from the group consisting of valine, isoleucine, leucine, tyrosine, cysteine, serine, threonine, aspartic acid, glutamic acid, tryptophan, methionine, phenylalanine, alanine, proline, glycine, glutamine, asparagine, lysine, and arginine;
(vii) the amino acid residue at the position corresponding to position 224 of SEQ ID NO: 1 is substituted and the amino acid residue at the position corresponding to position 224 of SEQ ID NO: 1 is an amino acid residue selected from the group consisting of isoleucine, leucine, valine, methionine, cysteine, tyrosine, serine, threonine, aspartic acid, glutamic acid, tryptophan, phenylalanine, proline, glycine, glutamine, asparagine, lysine, histidine, and arginine;
(viii) the amino acid residue at the position corresponding to position 257 of SEQ ID NO: 1 is substituted and the amino acid residue at the position corresponding to position 257 of SEQ ID NO: 1 is an amino acid residue selected from the group consisting of phenylalanine, leucine, isoleucine, cysteine, serine, threonine, aspartic acid, glutamic acid, tryptophan, methionine, valine, alanine, proline, glycine, glutamine, asparagine, lysine, histidine, and arginine;
(ix) the amino acid residue at the position corresponding to position 229 of SEQ ID NO: 1 is substituted and the amino acid residue at the position corresponding to position 229 of SEQ ID NO: 1 is an amino acid residue selected from the group consisting of phenylalanine, leucine, isoleucine, cysteine, serine, threonine, aspartic acid, glutamic acid, tryptophan, methionine, valine, alanine, proline, glycine, glutamine, asparagine, lysine, histidine, and arginine; or
(x) the amino acid residue at the position corresponding to position 158 of SEQ ID NO: 1 is substituted and the amino acid residue at the position corresponding to position 158 of SEQ ID NO: 1 is an amino acid residue selected from the group consisting of aspartic acid, glutamic acid, lysine, arginine, histidine, tyrosine, cysteine, serine, threonine, tryptophan, methionine, leucine, isoleucine, phenylalanine, alanine, proline, glycine, glutamine, and asparagine.

8. The luciferase mutant of Claim 7, wherein
(i) the amino acid residue at the position corresponding to position 221 of SEQ ID NO: 1 is substituted and the amino acid residue at the position corresponding to position 221 of SEQ ID NO: 1 is leucine;
(ii) the amino acid residue at the position corresponding to position 17 of SEQ ID NO: 1 is substituted and the amino acid residue at the position corresponding to position 17 of SEQ ID NO: 1 is aspartic acid, glutamic acid, or lysine;
(iii) the amino acid residue at the position corresponding to position 18 of SEQ ID NO: 1 is substituted and the amino acid residue at the position corresponding to position 18 of SEQ ID NO: 1 is leucine;
(iv) the amino acid residue at the position corresponding to position 75 of SEQ ID NO: 1 is substituted and the amino acid residue at the position corresponding to position 75 of SEQ ID NO: 1 is lysine;
(v) the amino acid residue at the position corresponding to position 110 of SEQ ID NO: 1 is substituted and the amino acid residue at the position corresponding to position 110 of SEQ ID NO: 1 is aspartic acid, glutamic acid, lysine, or arginine;
(vi) the amino acid residue at the position corresponding to position 223 of SEQ ID NO: 1 is substituted and the amino acid residue at the position corresponding to position 223 of SEQ ID NO: 1 is valine, isoleucine, or leucine;
(vii) the amino acid residue at the position corresponding to position 224 of SEQ ID NO: 1 is substituted and the amino acid residue at the position corresponding to position 224 of SEQ ID NO: 1 is isoleucine;
(viii) the amino acid residue at the position corresponding to position 257 of SEQ ID NO: 1 is substituted and the amino acid residue at the position corresponding to position 257 of SEQ ID NO: 1 is phenylalanine;
(ix) the amino acid residue at the position corresponding to position 229 of SEQ ID NO: 1 is substituted and the amino acid residue at the position corresponding to position 229 of SEQ ID NO: 1 is phenylalanine or leucine; or
(x) the amino acid residue at the position corresponding to position 158 of SEQ ID NO: 1 is substituted and the amino acid residue at the position corresponding to position 158 of SEQ ID NO: 1 is aspartic acid or glutamic acid.

9. The luciferase mutant of any one of Claims 1 to 8, wherein the luciferase before amino acid substitution comprises an amino acid sequence selected from the group consisting of (A) to (F):
(A) an amino acid sequence having at least 70%, 80%, or 90% amino acid sequence identity to SEQ ID NO: 10;
(B) with regard to the amino acid sequence of (A), additionally, sequence identity between the homologous region of SEQ ID NO: 1 and the homologous region of the luciferase before amino acid substitution is 90% or higher in the amino acid sequence;
(C) an amino acid sequence derived from the amino acid sequence of SEQ ID NO: 10 by substitution, deletion, or addition of one or several amino acids at a position other than the position corresponding to position 252 of SEQ ID NO: 1;
(D) with regard to the amino acid sequence of (C), additionally, an amino acid sequence derived from the amino acid sequence of SEQ ID NO: 1, 10, 69, 71, or 73 by substitution, deletion, or addition of one or several amino acids at positions other than positions corresponding to position 90, 262, 371, 41, 427, 53, 306, 312, 294, 221, 17, 18, 75, 110, 223, 224, 257, 229, or 158 of SEQ ID NO: 1;
(E) the amino acid sequence of SEQ ID NO: 10; and
(F) an amino acid sequence of luciferase of Luciola lateralis.

10. The luciferase mutant of Claim 9, wherein the luciferase before amino acid substitution comprises an amino acid sequence selected from the group consisting of (a) to (d):
(a) an amino acid sequence having 90% or higher amino acid sequence identity to SEQ ID NO: 10;
(b) an amino acid sequence derived from the amino acid sequence of SEQ ID NO: 1 by substitution, deletion, or addition of one or several amino acids at a position other than the position corresponding to position 252 of SEQ ID NO: 1;
(c) with regard to the amino acid sequence of (b), additionally, an amino acid sequence derived from the amino acid sequence of SEQ ID NO: 1 by substitution, deletion, or addition of one or several amino acids at positions other than positions corresponding to position 262, 371, 41, 427, 53, 306, 312, 252, 294, 221, 17, 18, 75, 110, 223, 224, 257, 229, or 158 of SEQ ID NO: 1; and
(d) the amino acid sequence of SEQ ID NO: 10 .

11. A polynucleotide encoding the luciferase mutant of any one of Claims 1 to 10.

12. A vector comprising the polynucleotide of Claim 11.

13. A host cell comprising the polynucleotide of Claim 11 or the vector of Claim 12.

14. A production method of a luciferase mutant having improved thermostability comprising a step of culturing the host cell of Claim 13.

15. A kit for detecting at least one of ATP, ADP, and AMP comprising the luciferase mutant of any one of Claims 1 to 10.

16. A method for detecting at least one of ATP, ADP, and AMP comprising using the luciferase mutant of any one of Claims 1 to 10.

17. A luciferase mutant comprising an amino acid sequence in which at least one amino acid residue at the position corresponding to position 252, 262, 294, 90, 371, 41, 427, 53, 306, 312, 221, 17, 18, 75, 110, 223, 224, 257, 229, or 158 of SEQ ID NO: 1 is substituted, wherein the luciferase after amino acid substitution has improved thermostability compared with that of the luciferase before amino acid substitution.

18. The luciferase mutant of Claim 17,
wherein
the position corresponding to position 252 of SEQ ID NO: 1 is substituted with leucine;
the position corresponding to position 262 of SEQ ID NO: 1 is substituted with tyrosine;
the position corresponding to position 294 of SEQ ID NO: 1 is substituted with leucine;
the position corresponding to position 90 of SEQ ID NO: 1 is substituted with tyrosine;
the position corresponding to position 371 of SEQ ID NO: 1 is substituted with leucine;
the position corresponding to position 41 of SEQ ID NO: 1 is substituted with valine;
the position corresponding to position 427 of SEQ ID NO: 1 is substituted with phenylalanine;
the position corresponding to position 53 of SEQ ID NO: 1 is substituted with leucine;
the position corresponding to position 306 of SEQ ID NO: 1 is substituted with phenylalanine;
the position corresponding to position 312 of SEQ ID NO: 1 is substituted with glutamic acid, lysine, or arginine;
the position corresponding to position 221 of SEQ ID NO: 1 is substituted with leucine;
the position corresponding to position 17 of SEQ ID NO: 1 is substituted with aspartic acid, glutamic acid, or lysine;
the position corresponding to position 18 of SEQ ID NO: 1 is substituted with leucine;
the position corresponding to position 75 of SEQ ID NO: 1 is substituted with lysine;
the position corresponding to position 110 of SEQ ID NO: 1 is substituted with aspartic acid, glutamic acid, lysine, or arginine;
the position corresponding to position 223 of SEQ ID NO: 1 is substituted with valine, isoleucine, or leucine;
the position corresponding to position 224 of SEQ ID NO: 1 is substituted with isoleucine;
the position corresponding to position 257 of SEQ ID NO: 1 is substituted with phenylalanine;
the position corresponding to position 229 of SEQ ID NO: 1 is substituted with phenylalanine or leucine; or
the position corresponding to position 158 of SEQ ID NO: 1 is substituted with aspartic acid or glutamic acid.
